(19) Europäisches Patentamt
European Patent Office
Office européen des brevets

(11) Publication number: 0 508 800 A1

# (12) EUROPEAN PATENT APPLICATION

(21) Application number: **92303197.5**

(22) Date of filing: **10.04.92**

(51) Int. Cl.[5]: **C07D 401/04,** A01N 43/50, C07D 491/04, C07D 495/04, A01N 43/40, A01N 43/90, A01N 43/42

(30) Priority: **11.04.91 GB 9107742**

(43) Date of publication of application: **14.10.92 Bulletin 92/42**

(84) Designated Contracting States: **AT BE CH DE DK ES FR GB GR IT LI LU MC NL PT SE**

(71) Applicant: **RHONE POULENC AGRICULTURE LTD.**
**Fyfield Road**
**Ongar, Essex CM5 OHW (GB)**

(72) Inventor: **Pearson, Christopher John**
**c/o Research Station, Rhone Poulenc Agric. Ltd.**
**Fyfield Road, Ongar, Essex CM5 OHW (UK)**
Inventor: **Cramp, Michael Colin**
**c/o Res. Station, Rhone Poulenc Agriculture Ltd.**
**Fyfield Road, Ongar, Essex CM5 OHW (UK)**
Inventor: **Luheshi, Abdul**
**c/o Rhone-Poulenc Agriculture Limited,**
**Fyfield Road, Ongar,Essex, CM5 OHW, (UK)**

(74) Representative: **Bentham, Stephen**
**J.A. Kemp & Co. 14 South Square Gray's Inn**
**London, WC1R 5LX (GB)**

(54) **New Herbicides.**

(57) Benzimidazolyl derivatives of formula

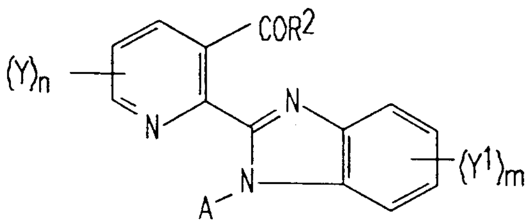

wherein Y represents :-

an optionally substituted alkyl, alkenyl or alkynyl group ; or an optionally substituted cycloalkyl group ; or a group -SR, -OR, halogen, aryl or aralkyl ; or 2 groups Y form a fused phenyl ring ;

$R^2$ represents a group -OH, $-NR^7R^8$ or -XM ;

A represents a group $-SO_2NR^7R^8$ ;

$Y^1$ represents an optionally substituted alkyl, alkenyl or alkynyl group ; or an optionally substituted cycloalkyl group ; or a group such as -SR, -OR, halogen, aryl, aralkyl, -O-aryl or $-NR^7R^8$ ;

n represents one or two ;

m represents an integer from 1 to 4 ;

R represents an optionally substituted alkyl group ; or an optionally substituted cycloalkyl group ;

$R^7$ and $R^8$ represent independently a group such as hydrogen, R, aryl or aralkyl ; X represents oxygen or sulphur ;

M represents an optionally substituted alkyl group ; or an optionally substituted cycloalkyl group ; or an aryl, aralkyl, alkenyl, alkynyl, or an imine group ; agriculturally acceptable salts thereof ;

the herbicidal compositions comprising these compounds and their application to crop protection is described.

EP 0 508 800 A1

This invention relates to novel compounds, processes for their preparation, compositions containing them and their use as herbicides.

The present invention provides benzimidazolyl derivatives of general formula I :-

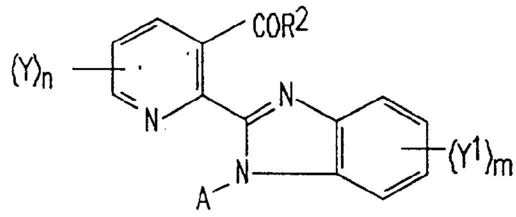

wherein

Y represent:-

a straight- or branched- chain alkyl, alkenyl or alkynyl group containing up to eight carbon atoms which is optionally substituted by one or more $R^1$ groups which may be the same or different; or

a cycloalkyl group containing from 3 to 6 carbon atoms optionally substituted by one or more $R^1$ groups which may be the same or different; or a group selected from -SR, -OR, halogen, aryl or aralkyl;

$Y^1$ represents:-

a straight- or branched- chain alkyl, alkenyl or alkynyl group containing up to eight carbon atoms which is optionally substituted by one or more $R^1$ groups which may be the same or different; or

a cycloalkyl group containing from 3 to 6 carbon atoms optionally substituted by one or more $R^1$ groups which may be the same or different; or a group selected from -SR, -OR, halogen, aryl, aralkyl, O-aryl or $-NR^7R^8$;

$R^2$ represents:-

a group -OH or $-NR^7R^8$;

or -X-M, where X represents oxygen or sulphur, and

M represents:-

a straight- or branched- chain alkyl group containing up to eight carbon atoms optionally substituted by one or more $R^1$ groups which may be the same or different; or

a cycloalkyl group containing from 3 to 6 carbon atoms optionally substituted by one or more $R^1$ groups which may be the same or different; or a group selected from aryl and aralkyl; or

a group 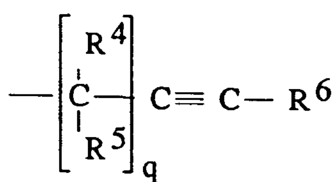 ; or

a group 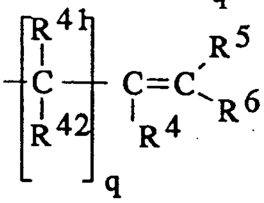 ; or

a group 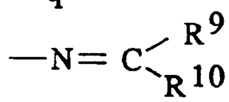 ;

A represents a group $-SO_2NR^7R^8$;

R represents:-

a straight- or branched- chain alkyl group containing up to eight carbon atoms optionally substituted by one or more groups $R^1$ which may be the same or different; or

a cycloalkyl group containing from 3 to 6 carbon atoms optionally substituted by one or more groups $R^3$ which may be the same or different;

$R^1$ represents:-

a cycloalkyl group containing from 3 to 6 carbon atoms; or

a group selected from $-OR^3$, $-SR^3$, halogen, $R^3$ or O-aryl;

$R^3$ represents a straight- or branched- chain alkyl group containing up to 6 carbon atoms optionally substituted by one or more halogen atoms which may be the same or different;

$R^4$, $R^{41}$, $R^{42}$ and $R^5$, which may be the same or different, each represent hydrogen or a straight- or branched-chain alkyl group containing up to 6 carbon atoms optionally substituted by one or more halogen atoms which may be the same or different; or aryl;

$R^6$ represents a group selected from $R^4$ or aralkyl;

$R^7$ and $R^8$, which may be the same or different, each represent:-

a hydrogen atom, or

a group selected from R, $-OR^3$, $-SR^3$, halogen, $R^3$, O-aryl, aryl or aralkyl; or $R^7$ and $R^8$ may form together with the nitrogen to which they are attached a heterocycle containing from 3 to 6 carbon atoms in the ring and zero, 1 or 2 additional heteroatoms in the ring selected from nitrogen, oxygen and sulphur;

$R^9$ and $R^{10}$, which may be the same or different, each represent:

a hydrogen atom, or

a straight- or branched- chain alkyl group containing up to eight carbon atoms optionally substituted by one or more groups, which may be the same or different, selected from halogen,-OR or $-S(O)_rR$, where r is zero, 1 or 2; or

a phenyl group optionally substituted by from one to four groups, which may be the same or different, selected from nitro, R, $-NR^4R^5$, halogen and $-S(O)_rR$; or

a 5 or 6 membered heterocycle containing from 3 to 5 carbon atoms in the ring and one or more heteroatoms in the ring selected from nitrogen, sulphur or oxygen, e.g. thienyl, furyl, piperidyl, thiazolyl, optionally substituted by one or more groups $R^1$ which may be the same or different;

'aryl' represents:-

a phenyl group optionally substituted by from 1 to 4 groups which may be the same or different selected from $-OR^3$, $-SR^3$, halogen or $R^3$; or

a 5 or 6 membered heterocycle containing from 3 to 5 carbon atoms in the ring and one or more heteroatoms in the ring selected from nitrogen, sulphur or oxygen, e.g. thienyl, furyl, piperidyl, thiazolyl; optionally substituted by one or more groups, which may be the same or different, selected from $-OR^3$, $-SR^3$, halogen or $R^3$;

'aralkyl' represents a group $-(CR^4R^5)_p$-aryl(e.g. benzyl);

m represents an integer from 1 to 4, the groups $Y^1$ being the same or different when m is greater than 1;

n represents one or two;

when n represents 2, two groups Y in the 5- and 6- positions of the pyridine ring together form an unsubstituted fused phenyl ring;

p represents one or two;

q represents one or two; and

where m represents an integer greater than 1, two groups $Y^1$ selected from -SR, -OR and straight- or branched- chain alkyl groups containing up to 8 carbon atoms optionally substituted by one or more groups $R^1$ which may be the same different, together in the 4,5 position or the 5,6 position can, with the carbon atoms to which they are attached, form an aliphatic ring having 5 or 6 atoms in the ring and having not more than 2 heteroatoms in the ring selected from oxygen and sulphur, e.g. 1,3-dioxolo[4,5-e] benzimidazole; 5,6-dihydrofuro[2,3-e] benzimidazole; 4,5 -dihydrofuro[3,2-e] benzimidazole; 4,5-dihydrothieno[3,2-e] benzimidazole;

and agriculturally acceptable salts thereof;

which possess valuable herbicidal properties.

Furthermore, in certain cases the substituents Y, $Y^1$, $R^2$ and A may give rise to optical isomerism and/or stereoisomerism. All such forms are embraced by the present invention.

By the term "agriculturally acceptable salts" is meant salts the cations or anions of which are known and accepted in the art for the formation of salts for agricultural or horticultural use. Preferably the salts are water-soluble.

Suitable salts formed by compounds of formula I which are acidic, i.e. compounds containing a carboxy group, with bases include alkali metal (e.g sodium and potassium) salts, alkaline earth metal (e.g. calcium and magnesium) salts, ammonium and amine (e.g diethanolamine, triethanolamine, octylamine, dioctylmethylamine and morpholine) salts.

Suitable acid addition salts, formed by compounds of general formula I containing an amino group, include salts with inorganic acids, for example hydrochlorides, sulphates, phosphates and nitrates and salts with inorganic acids, for example acetic acid. Preferred salts are those in which $R^2$ represents a group -XW, where W represents a cation selected from the alkali metals (e.g., Na, K, Li) or ammonium salts of formula -

$NR^{11}R^{12}R^{13}R^{14}$, where $R^{11}$, $R^{12}$, $R^{13}$ and $R^{14}$, which may be the same or different, each represent:-

a hydrogen atom, or

a straight- or branched- chain alkyl group containing up to 6 carbon atoms optionally substituted by -OH; or aralkyl, not more than two of the radicals $R^{11}$ to $R^{14}$ representing the aralkyl group.

It is to be understood that where reference is made in the present specification to the compounds of general formula I, such reference is intended to include salts where the context so permits.

Compounds of general formula (I) in which $R^2$ represents an alkoxy group are of particular utility in the preparation of other compounds of general formula (I), in particular those in which $R^2$ represents hydroxy.

A preferred class of compounds of general formula (I) are those wherein n represents one.

Preferred compounds of general formula (I) in which n represents 2 and two groups Y in the 5- and 6- positions of the pyridine ring together form an unsubstituted fused phenyl ring are those wherein:

A represents $-SO_2NMe_2$;

$Y^1$ represents:-

a halogen atom, a group -OR, or a straight- or branched- chain alkyl group containing up to 4 carbon atoms optionally substituted by one or more halogen atoms;

m represents one or two; and

R represents a straight- or branched- chain alkyl group containing up to 4 carbon atoms optionally substituted by one or more halogen atoms;

the other symbols being as hereinbefore defined; which possess advantageous properties, for example in the selective control of weed species in soya.

A particularly important class of compounds of general formula (I) because of their herbicidal properties are those in which:

(a) A represents $-SO_2NMe_2$; and/or

(b) $R^2$ represents -OH or -XM; and/or

(c) Y represents an alkyl group containing one or two carbon atoms optionally substituted by one or more halogen atoms, for example methyl, ethyl or trifluoromethyl; and/or

(d) $Y^1$ represents -OR, for example methoxy or ethoxy; or halogen, for example fluorine or chlorine; or $-NR^7R^8$; and/or

(e) m represents one or two; and/or

(f) $R^7$ and $R^8$, which may be the same or different, each represent a straight- or branched-chain alkyl group containing up to 4 carbon atoms optionally substituted by one or more atoms which may be the same or different selected from fluorine or chlorine; and/or (g) X represents oxygen; and/or

(h) M represents $-CH_2C{\equiv}CH$ or $CH_2CH{=}CH_2$; and/or

(i) the group or groups $Y^1$ occupy the 4 and/or 5 position of the benzimidazole ring; and/or

(j) n represents one;

the other symbols being as hereinbefore described.

A further preferred class of compounds of general formula (I) are those in which $Y^1$ represents a straight- or branched- chain alkyl group containing up to 4 carbon atoms.

A further preferred class of compounds of general formula (I) are those wherein:

A represents $-SO_2NMe_2$;

Y represents methyl or ethyl, or two groups Y in the 5- and 6- positions of the pyridine ring together with the carbon atoms to which they are attached form an unsubstituted fused phenyl ring;

$R^2$ represents -OH or -X-M; X represents oxygen;

M represents 2-propynyl, methyl or an isopropylammonium or potassium cation;

$Y^1$ represents chlorine, methyl, methoxy, ethoxy, n-propyloxy or isopropyloxy;

m represents one or two; and n represents one or two.

Particularly important compounds because of their herbicidal properties include the following:-

1. 2-propynyl 2-[1-(N,N-dimethylsulphamoyl)-4-methoxybenzimidazol-2-yl]-6-methylpyridine-3-carboxylate;
2. 2-propynyl 2-[1-(N,N-dimethylsulphamoyl)-4-ethoxybenzimidazol-2-yl]-6-methylpyridine-3-carboxylate;
3. 2-propynyl 2-[1-(N,N-dimethylsulphamoyl)-5-chlorobenzimidazol-2-yl]-6-methylpyridine-3-carboxylate;
4. 2-propynyl 2-[1-(N,N-dimethylsulphamoyl)-5(6)-methylbenzimidazol-2-yl]-6-methylpyridine-3-carboxylate;
5. 2-propynyl 2-[1-(N,N-dimethylsulphamoyl)-5(6)-methylbenzimidazol-2-yl]-5-methylpyridine-3-carboxylate;
6. 2-propynyl 2-[1-(N,N-dimethylsulphamoyl)-4-methylbenzimidazol-2-yl]-5-methylpyridine-3-carboxylate;

7. 2-propynyl 2-[1-(N,N-dimethylsulphamoyl)-5-chlorobenzimidazol-2-yl]-5-methylpyridine-3-carboxylate;
8. 2-[1-(N,N-dimethylsulphamoyl)-4-methoxybenzimidazol-2-yl]-6-methylpyridine-3-carboxylic acid;
9. 2-[1-(N,N-dimethylsulphamoyl)-5-fluorobenzimidazol-2-yl]-6-methylpyridine-3-carboxylic acid;
10. 2-[1-(N,N-Dimethylsulphamoyl)-4-ethoxybenzimidazol-2-yl]-6-methylpyridine-3-carboxylic acid;
11. 2-[1-(N,N-Dimethylsulphamoyl)-4,5-dimethylbenzimidazol-2-yl]-6-methylpyridine-3-carboxylic acid;
12. 2-[1-(N,N-Dimethylsulphamoyl)-5-chlorobenzimidazol-2-yl]-6-methylpyridine-3-carboxylic acid;
13. 2-[1-(N,N-dimethylsulphamoyl)-5(6)-methylbenzimidazol-2-yl]-6-methylpyridine-3-carboxylic acid;
14. 2-[1-(N,N-dimethylsulphamoyl)-4-methylbenzimidazol-2-yl]-6-methylpyridine-3-carboxylic acid;
15. 2-[1-(N,N-dimethylsulphamoyl)-4-*n*-propyloxy-benzimidazol-2-yl]-6-methylpyridine-3-carboxylic acid;
16. 2-[1-(N,N-dimethylsulphamoyl)-4-*i*-propyloxybenzimidazol-2-yl]-6-methylpyridine-3-carboxylic acid;
17. 2-[1-(N,N-dimethylsulphamoyl)-4-chlorobenzimidazol-2-yl]-6-methylpyridine-3-carboxylic acid;
18. 2-[1-(N,N-dimethylsulphamoyl)-5(6)-methylbenzimidazol-2-yl]-5-ethylpyridine-3-carboxylic acid;
19. 2-[1-(N,N-dimethylsulphamoyl)-5(6)-methoxybenzimidazol-2-yl]-5-ethylpyridine-3-carboxylic acid;
20. 2-[1-(N,N-dimethylsulphamoyl)-5-chlorobenzimidazol-2-yl]-5-ethylpyridine-3-carboxylic acid;
21. 2-[1-(N,N-dimethylsulphamoyl)-4-methylbenzimidazol-2-yl]-5-ethylpyridine-3-carboxylic acid;
22. 2-[1-(N,N-dimethylsulphamoyl)-4-methylbenzimidazol-2-yl]-5-ethylpyridine-3-carboxylic acid;
23. 2-[1-(N,N-dimethylsulphamoyl)-4-ethoxybenzimidazol-2-yl]-5-ethylpyridine-3-carboxylic acid;
24. 2-[1-(N,N-dimethylsulphamoyl)-4-n-propyloxy-benzimidazol-2-yl]-5-ethylpyridine-3-carboxylic acid;
25. 2-[1-(N,N-dimethylsulphamoyl)-4-methoxybenzimidazol-2-yl]-5-methylpyridine-3-carboxylic acid;
26. 2-[1-(N,N-dimethylsulphamoyl)-5(6)-methylbenzimidazol-2-yl]-5-methylpyridine-3-carboxylic acid;
27. 2-[1-(N,N-dimethylsulphamoyl)-4-methylbenzimidazol-2-yl]-5-methylpyridine-3-carboxylic acid;
28. 2-[1-(N,N-dimethylsulphamoyl)-5-chlorobenzimidazol-2-yl]-5-methylpyridine-3-carboxylic acid;
29. 2-[1-(N,N-dimethylsulphamoyl)-4-*n*-propyloxybenzimidazol-2-yl]-5-methylpyridine-3-carboxylic acid;
30. 2-[1-(N,N-dimethylsulphamoyl)-4-ethoxybenzimidazol-2-yl]-5-methylpyridine-3-carboxylic acid;
31. 2-[1-(N,N-dimethylsulphamoyl)-4-*i*-propyloxybenzimidazol-2-yl]-5-methylpyridine-3-carboxylic acid;
32. 2-[1-(N,N-dimethylsulphamoyl)-4-*i*-propyloxybenzimidazol-2-yl]-5-ethylpyridine-3-carboxylic acid;
33. isopropylammonium 2-[1-(N,N-dimethylsulphamoyl)-5-chlorobenzimidazol-2-yl]-6-methylpyridine-3-carboxylate;
34. potassium 2-[1-(N,N-dimethylsulphamoyl)-4-methylbenzimidazol-2-yl]-5-methylpyridine-3-carboxylate;
35. isopropylammonium 2-[1-(N,N-dimethylsulphamoyl)-4-methyl-benzimidazol-2-yl]-5-methylpyridine-3-carboxylate;
36 potassium 2-[1-(N,N-dimethylsulphamoyl)-5-chlorobenzimidazol-2-yl]-5-methylpyridine-3-carboxylate;
37. isopropylammonium 2-[1-(N,N-dimethylsulphamoyl)-5-chlorobenzimidazol-2-yl]-5-methylpyridine-3-carboxylate;
38. 2-[1-(N,N-dimethylsulphamoyl)-4-methoxylbenzimidazol-2-yl]quinoline-3-carboxylic acid;
39. 2-[1-(N,N-dimethylsulphamoyl)-4-ethoxylbenzimidazol-2-yl]quinoline-3-carboxylic acid;
40. 2-[1-(N,N-dimethylsulphamoyl)-4,5-dimethylbenzimidazol-2-yl]quinoline-3-carboxylic acid;
41. 2-[1-(N,N-dimethylsulphamoyl)-5(6)-methylbenzimidazol-2-yl]quinoline-3-carboxylic acid;
42. 2-[1-(N,N-dimethylsulphamoyl)-4-*i*-propyloxybenzimidazol-2-yl]quinoline-3-carboxylic acid;
43. 2-[1-(N,N-dimethylsulphamoyl)-4-methylbenzimidazol-2-yl]quinoline-3-carboxylic acid;
44. 2-[1-(N,N-dimethylsulphamoyl)-5-chlorobenzimidazol-2-yl]quinoline-3-carboxylic acid;
45. 2-propynyl 2-[1-(N,N-dimethylsulphamoyl)-5(6)-methylbenzimidazol-2-yl]quinoline-3-carboxylate.
46. methyl 2-[1-(N,N-dimethylsulphamoyl)-4-methoxybenzimidazol-2-yl]-6-methylpyridine-3-carboxylate;
47. methyl 2-[1-(N,N-dimethylsulphamoyl)-5-chlorobenzimidazol-2-yl]-6-methylpyridine-3-carboxylate;
48. methyl 2-[1-(N,N-dimethylsulphamoyl)-4-methoxybenzimidazol-2-yl]quinoline-3-carboxylate; and
49. methyl 2-[1-(N,N-dimethylsulphamoyl)-4-ethoxybenzimidazoyl-2-yl]quinoline-3-carboxylate.

The numbers 1 to 49 are assigned these compounds hereafter.

The compounds of general formula I can be prepared by the application or adaptation of known methods (i.e. methods heretofore used or described in the chemical literature), for example as hereinafter described.

In the following description where symbols appearing in formulae are not specifically defined, it is to be understood that they are "as hereinbefore defined" in accordance with the first definition of each symbol in this specification.

It is to be understood that, in the description of the following processes, sequences may be performed in different orders and that suitable protecting groups may be required to achieve the compounds sought.

According to a feature of the present invention compounds of formula (I) in which A is the $SO_2NR^7R^8$ group and $R^2$ is a -XM or $-NR^7R^8$ radical can be prepared by reacting a sulphamoyl chloride of the formula $ClSO_2NR^7R^8$ with a compound of formula (Ia):

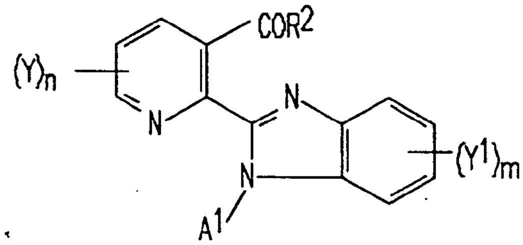

Ia

in which $A^1$ is the hydrogen atom and $R^2$ is an -XM or $-NR^7R^8$ radical, in the presence of an acid acceptor such as potassium carbonate, triethylamine, 1,8-diazabicyclo[5.9.0]undec-7-ene or sodium hydride, preferably in an anhydrous medium using an aprotic polar solvent, for example, ethers (such as THF) or nitriles, at a temperature which is generally between 25 °C and the reflux temperature of the solvent.

According to a further feature of the present invention compounds of formula (I) where $R^2$ is -XW in which X represents oxygen may be prepared from compounds of formula (I) where $R^2$ is -OH by reaction with the corresponding base.

According to a further feature of the present invention compounds of the formula (I) in which $R^2$ represents -OH, may be prepared by the hydrolysis of compounds of formula (I) in which $R^2$ represents the group -XM using an inorganic base, for example lithium hydroxide, in a mixture of water and an alcohol, for example methanol, at a temperature between 0 °C and 25 °C.

Intermediates in the preparation of compounds of general formula I are prepared by the application or adaptation of known methods. Compounds of formula (Ia) in which $A^1$ is the hydrogen atom and $R^2$ is a radical -XM or $-NR^7R^8$ may be prepared by reacting a compound of formula (II):

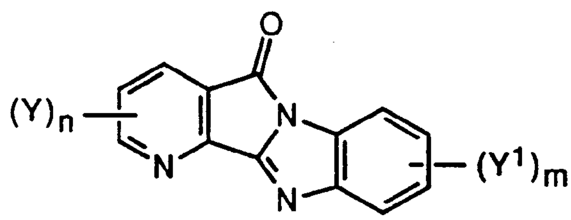

(II)

with an alkali metal alcoholate or alkaline earth metal alcoholate of the formula XMM′, wherein M′ represents an alkali metal or alkaline earth metal cation, in an aprotic solvent and at a temperature between 0 °C and the boiling point of the solvent, or with an alcohol, thiol or oxime of the formula H-XM, or with an amine of the formula $HNR^7R^8$. The reaction with H-XM or $HNR^7R^8$ is generally performed in a polar organic solvent in the presence of an acid acceptor such as pyridine or triethylamine.

Compounds of formula (Ia) in which $A^1$ represents the hydrogen atom and $R^2$ is a radical -XM or $NR^7R^8$ may be prepared by the reaction of the compound of formula (III):

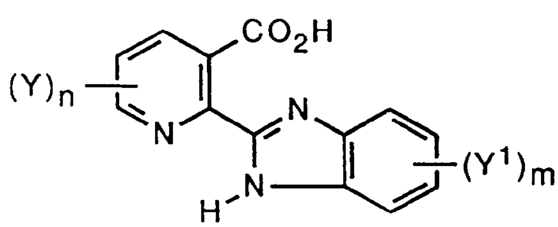

(III)

with an alcohol, thiol or oxime of formula H-XM or an amine of formula $HNR^7R^8$ in the presence of a coupling

reagent, for example

N,N-dicyclohexylcarbodiimide, in the presence of an insert solvent such as dichloromethane and at a temperature between 0 °C and the reflux temperature of the solvent.

Compounds of formula (Ia) in which $A^1$ is the hydrogen atom and $R^2$ is a radial -XM in which X is the oxygen atom and M is as hereinbefore defined excluding the group $-N=CR^9R^{10}$, may be prepared by reacting a compound of formula (III) with a compound of formula H-OM in the presence of gaseous HCl with the compound of formula H-OM also acting as a solvent following a well known esterification process.

Compounds of the formula (Ia), where $A^1$ is the hydrogen atom and $R^2$ is the radical -XM, where X represents the oxygen atom and M is as hereinbefore defined excluding the group $-N=CR^9R^{10}$, may be prepared by heating a compound of formula (IIIa):

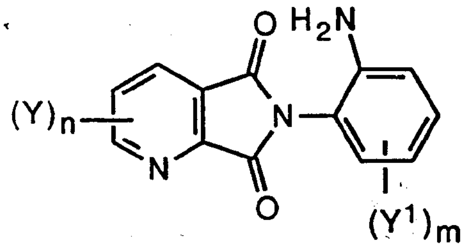

(IIIa)

in a high boiling compound of formula H-OM, where M has the abovementioned meaning, for example ethoxyethanol at temperatures between 50 °C and the reflux temperature of the solvent.

Compounds of formula (II) can be obtained by reacting an anhydride of formula (IV) with a 1,2-phenylenediamine of formula (V):

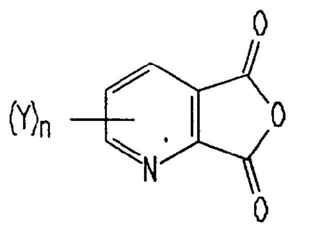

(IV)

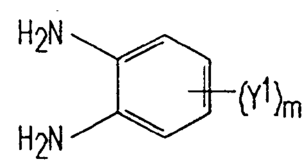

(V)

by heating at temperatures between 110 and 190°C for between 1 and 3 hours, either in the absence of a solvent or in a solvent such as xylene, dichlorobenzene, or acetic acid, followed by the addition of acetic anhydride and continued heating at a temperature between 70°C and the boiling point of the solvent.

Compounds of formula (III) may be prepared by the cyclisation of the compounds of formula (VI):

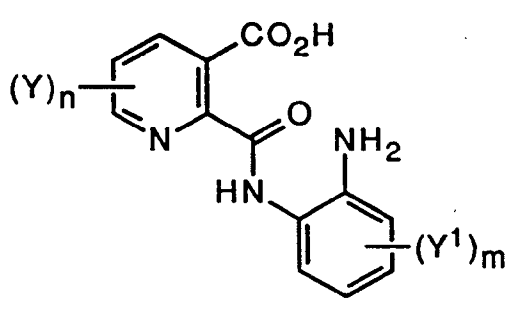

(VI).

The reaction may be carried out in an organic solvent such as ethoxyethanol under reflux. Compounds of

formula (VI) may be obtained by the reaction of an anhydride of formula (IV) with a 1,2-phenylene-diamine of formula (V) in an inert organic solvent, for example chloroform at temperatures between 0°C and the boiling point of the solvent. Compounds of formula (VI) may also be obtained by reaction of compounds of formula (VII)

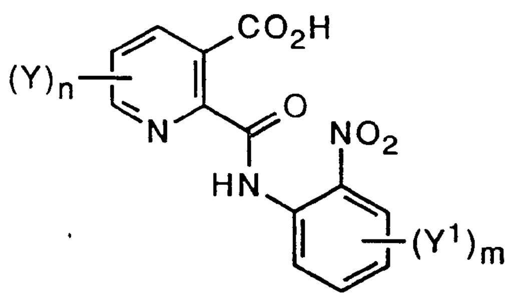

(VII).

The reaction may be carried out either in an aqueous basic or an organic solution, in the presence of hydrogen and of a hydrogenation catalyst for example platinum oxide, at a temperature between 20 and 60°C.

Compounds of the formula (VII) may be obtained by the reaction of an anhydride of formula (IV) with a 2-nitroaniline of the formula (VIII):

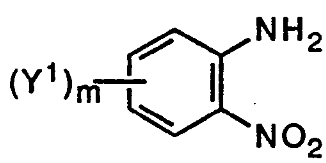

(VIII)

in an organic solvent, for example chloroform or tetrahydrofuran, at a temperature between 20°C and the boiling point of the solvent.

Compounds of formula (IIIa) may be prepared by the reduction of a compound of formula (IX):

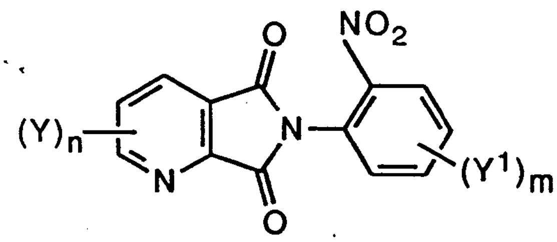

(IX)

in the presence of an aprotic polar solvent, for example THF or dioxan, or in a polar protic solvent, for example methanol in the presence of hydrogen and a hydrogenation catalyst such as palladium on charcoal and at a temperature between 20 and 60 °C.

Compounds of formula (IX) in which n represents one may be prepared by the oxidation of a dihydropyridine of formula (X):

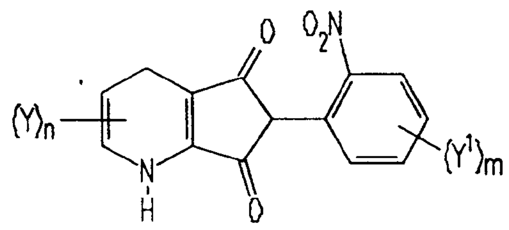

(X)

wherein n represents one, in the presence of an oxidising agent such as manganese(VI) oxide in an organic solvent such as acetic at a temperature between 20 and 80 °C. Alternatively, oxidation can be accomplished by using sulphur in an inert hydrocarbon solvent, for example toluene at the reflux temperature of the solvent. Compounds of formula (X) in which n represents one can be obtained by the reaction between an azabutadiene of formula (XI) and a maleimide of formula (XII):

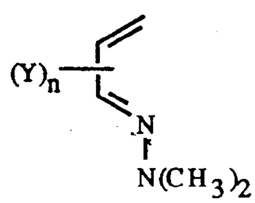

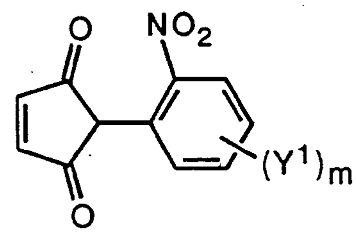

(XI) (XII)

wherein n represents one, in a polar aprotic solvent, for example acetonitrile at a temperature between 0 and 40°C and subsequently treating the reaction mixture with silica gel.

The maleimide of formula (XII) may be prepared by the reaction of a 2-nitroaniline of formula (VIII) with maleic anhydride in an organic solvent, for example chloroform and at the reflux temperature of the solvent, followed by the cyclisation of the intermediate of formula (XIII) by heating in acetic acid at reflux temperature in the presence of sodium acetate:

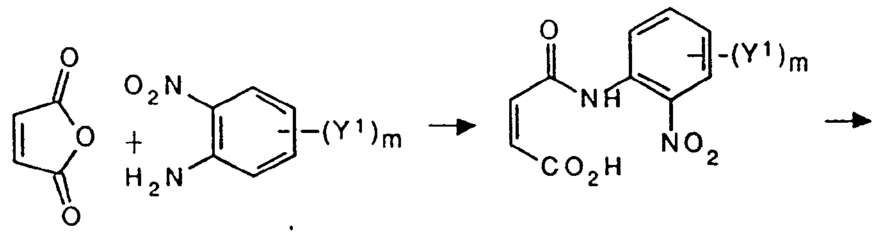

(XII)

(VIII) (XIII).

Azabutadienes of formula (XI) can be obtained as described by Waldner *et al.*, Helvetica Chimica Acta, 1988, 71, 493.

According to a further feature of the present invention compounds of general formula (I) in which m represents 1 or 2, one of the groups $Y^1$ represents a group -OR which occupies the 4- or 5- position of the benzimidazole ring, $R^2$ represents a group -X-M wherein X is oxygen and M represents a straight- or branched- chain alkyl group containing up to 8 carbon atoms optionally substituted by one or more $R^1$ groups which may be the same or different, or a cycloalkyl group containing from 3 to 6 carbon atoms optionally substituted by one or more $R^1$ groups which may be the same or different and the group Y is as hereinbefore defined excluding an alkenyl or alkynyl group or chlorine, bromine or iodine, may be prepared by the reaction of a hydroxybenzimidazole of formula (XIV):

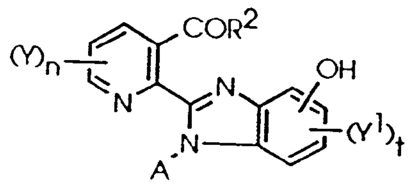

(XIV)

wherein t is 0 or 1 and the hydroxy group occupies the 4- or 5 position of the benzimidazole ring, with a compound R-L where L represents a leaving group, for example the tosyl group or a halogen atom (e.g. Cl, Br, I), to convert the hydroxy group in the benzimidazole ring into a group -OR. The reaction is generally carried out in the presence of a base such as potassium carbonate in an inert organic solvent, for example acetone or DMF, at temperatures between 0°C and the reflux temperature of the solvent.

The compounds of formula (XIV) can be prepared by the hydrogenolysis of a benzyloxybenzimidazole of formula (XV):

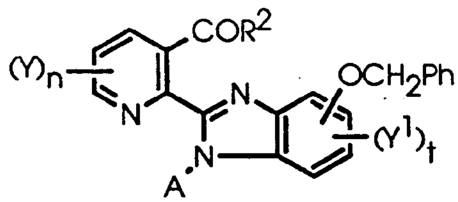

(XV)

in a protic solvent such as methanol in the presence of hydrogen or a hydrogen donor such as 1,4-cyclohexadiene and of a hydrogenation catalyst for example palladium on charcoal. The reaction is generally carried out at room temperature and at atmospheric pressure. The compounds of formula (XV) can be prepared by the application of methods hereinbefore described.

The 1,2-phenylenediamines of general formula (V) may be obtained by the reduction of 2-nitroanilines of formula (VIII) or of dinitrobenzenes of formula (XVI):

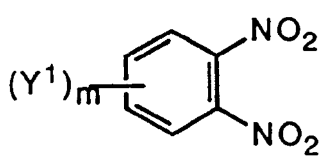

(XVI).

The reaction is carried out in a polar protic solvent such as methanol in the presence of hydrogen and of a hydrogenation catalyst such as palladium on charcoal at a temperature between 20 and 60 °C, or in hydrochloric acid in the presence of stannous chloride at a temperature between 40 and 90 °C.

Diamines of the formula (V) in which m represents 1 and the group $Y^1$ is an hereinbefore defined but does not represent a cycloalkyl group, and in which $Y^1$ occupies the 3- position of the benzene ring, can be prepared by the reduction of 2,1,3-benzoxadiazoles of formula (XVII):

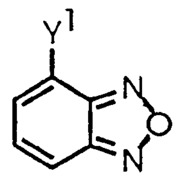

(XVII).

The reduction can be carried out in a polar protic solvent such as methanol in the presence of hydrogen and of a hydrogenation catalyst as for example, palladium on charcoal, at a temperature between 20 °C and the boiling point of the solvent, or in an aqueous methanol mixture at reflux in the presence of hydrochloric acid and finely divided iron.

Compounds of the formula (XVII) in which $Y^1$ represents a group -OR, -SR or $-NR^7R^8$ can be prepared by the reaction of 4-fluoro-2,1,3-benzoxadiazole of formula (XVIII):

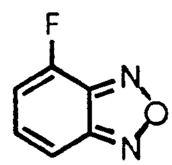

(XVIII)

with an alcohol H-OR, a thiol H-SR or an amine $H-NR^7R^8$. The reaction is typically carried out in acetone in the presence of a base such as potassium carbonate and at a temperature which is between 0°C and the reflux temperature of the solvent.

Alternatively preparation of compounds of the formula (XVII) in which $Y^1$ is a group -SR, may be prepared by the reaction of a compound of formula (XVIII) with a thiolate M'SR where M' is an alkali metal or alkaline earth metal cation, in methanol at a temperature between 20 °C and reflux. Conversion of a 4-fluoro-2,1,3-benzoxadiazole of formula (XVIII) into a compound of formula (XVII) in which $Y^1$ is an -OR group is conveniently carried out by reacting the former with an alcoholate M'-OR, with the corresponding alcohol H-OR as the reaction solvent at a temperature between 20 °C and the boiling point of the alcohol.

Compounds of formula (XVII) may also be prepared by the reaction of 2,1,3-benzoxadiazolide salts of formula (XIX):

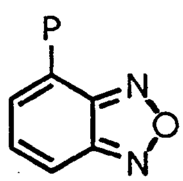

(XIX)

wherein P represents a cation selected from Na, K, Li, with an electrophilic reagent such as dialkyldisulphides of the formula RS-SR, for example dimethyl disulphide, or aldehydes for example acetaldehyde, or alkyl halides for example ethyl iodide, or imminium salts for example dimethyl imminium chloride. The reaction is performed in an aprotic solvent such as tetrahydrofuran at a temperature of -78 °C.

The 2,1,3-benzoxadiazolide salts of formula (XIX) may be prepared *in situ* by the reaction of 2,1,3-benzoxadiazole with an organic alkali base, for example lithium diisopropylamide in an aprotic solvent such as tetrahydrofuran at a temperature of approximately -78 °C.

4-Fluoro-2,1,3-benzoxadiazole of formula (XVIII) can be prepared by the method described by L. Di Nunno *et al.* in Journal of the Chemical Society (C), 1433, 1970.

2,1,3-benzoxadiazole can be prepared prepared according to the procedure of A. G. Green and F.M. Rowe, in Journal of the Chemical Society, 2452, **101**, 1912.

The 2-alkoxy-6-nitroanilines of formula (VIII) where m=1 and $Y^1$ is the group -OR may be prepared by the alkylation of an aminonitrophenol of formula (XX):

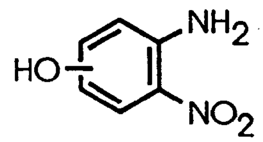

(XX)

with an alkylating agent R-halogen (where the halogen is for example Cl, Br, I) in the presence of a base, for example potassium carbonate, in an inert solvent, for example acetone or DMF and at temperatures between 0 °C and the reflux temperature of the solvent.

Nitroanilines of the formula (VIII) wherein m represents 1 and the group $Y^1$ occupies the 3-position of the benzene ring may be prepared by the hydrolysis of acetanilides of the formula (XXI):

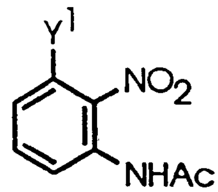

(XXI).

The hydrolysis is generally carried in an aqueous solution of sodium hydroxide in an alcoholic solvent such as ethanol at a temperature between 25 °C and the reflux temperature of the solvent.

Acetanilides of formula (XXI) wherein $Y^1$ represents -OR, -SR, or -$NR^7R^8$ may be prepared by the displacement of the 3-nitro group in 2,3-dinitroacetanilide with alcohols H-OR, thiols H-SR, or amines $HNR^7R^8$. The reaction of 2,3-dinitroacetanilide with alcohols is usually accomplished by using the alcohol H-OR as the solvent in the presence of at least one equivalent of the corresponding alcoholate Z-OR wherein Z is selected from Na, K, Li, and at a temperature between 20 °C and the boiling point of the alcohol. The reaction of 2,3-dinitroacetanilide with thiols is conveniently carried out in acetone in the presence of a base such as potassium carbonate and at a temperature between 0°C and reflux. Where 2,3-dinitroacetanilide is reacted with amines the reaction is typically carried out in a solvent such as ethanol at reflux.

2,3-Dinitroacetanilide may be prepared by using the method described by F.L. Grennwood *et al.*, in Journal of Organic Chemistry, 797, **20**, 1955.

2-Nitroanilines of general formula (VIII), dinitrobenzenes of formula (XVI) and anhydrides of formula (IV) are known or may be prepared by the application or adaption of known methods.

The following Examples illustrate the preparation of compounds of general formula (I) and the Reference Examples illustrate the preparation of intermediates. Unless otherwise stated percentages are by weight.

## EXAMPLE 1

Preparation of methyl 2-(1-N,N-dimethylsulfamoyl-4-methoxybenzimidazol-2-yl)-6-methylpyridine-3-carboxylate, compound 46.

A suspension of methyl 2-(4-methoxy-1*H*-benzimidazol-2-yl)-6-methylpyridine-3-carboxylate (4.6g) and anhydrous potassium carbonate (4.3g) in acetonitrile was stirred at reflux for 2 hours. Dimethylsulfamoyl chloride (16g) was added to the cooled reaction mixture, which was then stirred and heated at reflux for 6 hours. The cooled reaction mixture was evaporated and the residue dissolved in dichloromethane, washed with water, dried over magnesium sulphate and evaporated. The residue was crystallised from a mixture of ethyl acetate and hexane (1:1) to give the title compound (2.5g) as a yellow solid, m.p. 165-167°C (Yield: 41 %).

By proceeding in a similar manner, the following compounds were obtained from the appropriate starting

materials and are represented by the general formula:

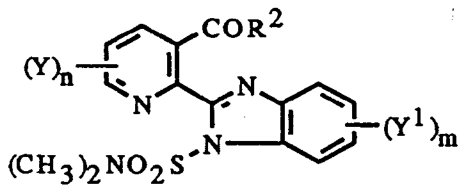

| Cpd. No | n | Y | m | $Y^1$ | $R^2$ | m.p./ C |
|---|---|---|---|---|---|---|
| 1 | 1 | 6-$CH_3$ | 1 | 4-$OCH_3$ | $OCH_2C{\equiv}CH$ | 156-158 |
| - | 1 | 6-$CH_3$ | 1 | 5-F | $OCH_3$ | 184-186 |
| - | 1 | 6-$CH_3$ | 1 | 4-$OC_2H_5$ | $OCH_3$ | 144-146 |
| 2 | 1 | 6-$CH_3$ | 1 | 4-$OC_2H_5$ | $OCH_2C{\equiv}CH$ | 150-151 |
| - | 1 | 6-$CH_3$ | 1 | 4,5-$(CH_3)_2$ | $OCH_3$ | nmr (a) |
| 47 | 1 | 6-$CH_3$ | 1 | 5-Cl | $OCH_3$ | 170-172 |
| 3 | 1 | 6-$CH_3$ | 1 | 5-Cl | $OCH_2C{\equiv}CH$ | 157.5-159 |
| - | 1 | 6-$CH_3$ | 1 | 5(6)-$CH_3$ | $OCH_3$ | 170.4-173 |
| 4 | 1 | 6-$CH_3$ | 1 | 5(6)-$CH_3$ | $OCH_2C{\equiv}CH$ | 136.5-138.5 |
| - | 1 | 6-$CH_3$ | 1 | 4-$CH_3$ | $OCH_3$ | 172-176 |
| - | 1 | 6-$CH_3$ | 1 | 4-O*n*-$C_3H_7$ | $OCH_3$ | not isolated |
| - | 1 | 6-$CH_3$ | 1 | 4-O*i*-$C_3H_7$ | $OCH_3$ | not isolated |
| - | 1 | 6-$CH_3$ | 1 | 4-Cl | $OCH_3$ | 222-224 |

| Cpd. No | n | Y | m | $Y^1$ | $R^2$ | m.p./ C |
|---|---|---|---|---|---|---|
| - | 1 | 5-$C_2H_5$ | 1 | 5(6)-$CH_3$ | $O(CH_2)_2OEt$ | oil, nmr (b) |
| - | 1 | 5-$C_2H_5$ | 1 | 5(6)-$OCH_3$ | $O(CH_2)_2OEt$ | gum, nmr (c) |
| - | 1 | 5-$C_2H_5$ | 1 | 5-Cl | $OCH_3$ | 159-160 |
| - | 1 | 5-$C_2H_5$ | 1 | 4-$CH_3$ | $OCH_3$ | nmr (d) |
| - | 1 | 5-$C_2H_5$ | 1 | 4-$OCH_3$ | $OCH_2C{\equiv}CH$ | gum, nmr (e) |
| - | 1 | 5-$C_2H_5$ | 1 | 4-$OC_2H_5$ | $OCH_3$ | 178-180 |
| - | 1 | 5-$C_2H_5$ | 1 | 4-O*n*-$C_3H_7$ | $OCH_3$ | 185-188 |
| - | 1 | 5-$CH_3$ | 1 | 4-$OCH_3$ | $OCH_2C{\equiv}CH$ | glass, nmr (f) |
| 5 | 1 | 5-$CH_3$ | 1 | 5(6)-$CH_3$ | $OCH_2C{\equiv}CH$ | 120-128 |
| 6 | 1 | 5-$CH_3$ | 1 | 4-$CH_3$ | $OCH_2C{\equiv}CH$ | 122-124 |
| 7 | 1 | 5-$CH_3$ | 1 | 5-Cl | $OCH_2C{\equiv}CH$ | 153-154 |
| - | 1 | 5-$CH_3$ | 1 | 4-O*n*-$C_3H_7$ | $OCH_3$ | 146-148 |
| - | 1 | 5-$CH_3$ | 1 | 4-$OC_2H_5$ | $OCH_3$ | nmr (g) |
| - | 1 | 5-$CH_3$ | 1 | 4-O*i*-$C_3H_7$ | $OCH_3$ | glass, nmr (h) |
| - | 1 | 5-$C_2H_5$ | 1 | 4-O*i*-$C_3H_7$ | $OCH_3$ | glass, nmr (h1) |

$H^1$ NMR

(a) ($CDCl_3$) d= 2.4 (3H, s), 2.55 (3H, s), 2.65 (3H, s), 2.85 (6H, s), 3.65 (3H, s), 7.2 (1H, d), 7.35 (1H, d), 8.3 (1H, d) ppm.

(b) ($CDCl_3$) d= 1.0 (3H, t), 1.3 (3H,t), 2.75 (6H, s), 3.15 (2H, m), 3.2 (2H, m), 3.8 (3H, d), 4.2 (2H, m), 6.9 (1H, m), 7.3 (1H, dd), 7.7 (1H, dd), 8.2 (1H, s), 8.6 (1H, s) ppm.

(c) ($CDCl_3$) d= 1.00 (3H, m), 1.3 (3H, m), 2.0 (2H, d), 2.5 (3H, d), 2.7(6H, s), 3.1 (2H, m), 3.2 (2H, m), 4.15 (2H, m), 7.15 (1H, m), 7.55 (1H, dd), 7.7 (1H, dd), 8.2 (1H, s), 8.6 (1H, s) ppm.

(d) ($CDCl_3$) d= 1.35 (3H, t), 2.65 (3H, s), 2.8 (6H, s), 2.85 (2H, q), 3.7 (3H, s), 7.2 (1H, d), 7.3 (1H, t), 7.75 (1H, d), 8.25 (1H, d), 8.65 (1H, d)

(e) ($CDCl_3$) d= 1.35 (3H, t), 2.20 (1H, t), 2.8 (2H, q), 2.88 (6H, s), 4.00 (3H, s), 4.65 (2H, d), 6.83 (1H, d), 7.38 (1H, s), 7.80 (1H, t), 7.50 (1H, d), 8.27 (1H, d), 8.55 (1H,d) ppm.

(f) ($CDCl_3$) d= 2.2 (1H, t), 2.5 (3H,s), 2.85 (6H, s), 4.0 (3H, s), 4.7 (2H, d), 6.8 (1H, d), 7.3 (1H, d), 7.5 (1H, d), 8.25 (1H, d), 8.65 (1H,d)

(g) (DMSO-$d_6$) d=1.4 (3H, t), 2.5 (3H, s), 2.8 (6H, s), 3,65 (3H, s), 4.25 (2H, q), 6.95 (1H, d), 7.35 (1H, t), 7.45 (1H, d), 8.3 (1H, d), 8.75 (1H, d)

(h) ($CDCl_3$) d= 1.43 (3H, d), 2.35 (6H, s), 2.5 (3H, s), 3.65 (3H,s), 4.90 (1H, m), 6.83 (1H, d), 7.28 (2H, m), 7.47 (1H, d), 8.25 (1H, s), 8.12 (1H, s) ppm.

(h1) (DMSO-$d_6$) d=1.30 (6H, m), 2.83 (6H, s), 3.70 (3H, s), 4.95 (1H, m), 6.95 (1H, d), 7.35 (1H, t), 7.40 (1H, d), 8.30 (1H, d), 8.78 (1H, d).

**EXAMPLE 2**

Preparation of 2-[1-N,N-dimethylsulphamoyl-4-methoxybenzimidazol-2-yl]-6-methylpyridine-3-carboxylic acid, compound 8.

A solution of LiOH (0.34g) in water (3.2g) in water (3.2 ml) was added to a suspension of methyl 2-[1-N,N-dimethylsulphamoyl)-4-methoxybenzimidazol-2-yl]-6-methylpyridine-3-carboxylate(2.0g) in methanol (11 ml) and the resulting mixture was stirred at room temperature for 12 hours. The methanol was evaporated and the residue taken up in water, washed with dichloromethane and the aqueous layer was acidified with 2M hydrochloric acid. The resulting white precipitate was collected by filtration, washed with water and dried to give the title compound (1.8g) as a white solid, m.p. 211-241°C (Yield: 94%).

By proceeding in a similar manner the following compounds were obtained from the appropriate starting materials and are represented by the formula:

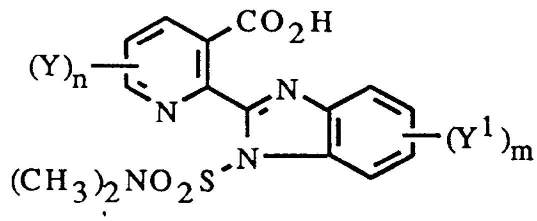

| Cpd. No | n | Y | m | Y1 | m.p./ C |
|---|---|---|---|---|---|
| 9 | 1 | 6-$CH_3$ | 1 | 5-F | 242-244 |
| 10 | 1 | 6-$CH_3$ | 1 | 4-$OC_2H_5$ | 148-151 |
| 11 | 1 | 6-$CH_3$ | 2 | 4,5-di$CH_3$ | 201-212 |
| 12 | 1 | 6-$CH_3$ | 1 | 5-Cl | 180-182 |
| 13 | 1 | 6-$CH_3$ | 1 | 5(6)-$CH_3$ | 203-213 |
| 14 | 1 | 6-$CH_3$ | 1 | 4-$CH_3$ | 155.5-156.7 |
| 15 | 1 | 6-$CH_3$ | 1 | 4-O*n*-$C_3H_7$ | 161 dec. |
| 16 | 1 | 6-$CH_3$ | 1 | 4-O*i*-$C_3H_7$ | 141-143 |
| 17 | 1 | 6-$CH_3$ | 1 | 4-Cl | 282-284 |
| 18 | 1 | 5-$C_2H_5$ | 1 | 5(6)-$CH_3$ | 105-107 |
| 19 | 1 | 5-$C_2H_5$ | 1 | 5(6)-$OCH_3$ | 112-115 |
| 20 | 1 | 5-$C_2H_5$ | 1 | 5-Cl | 144-146.5 |
| 21 | 1 | 5-$C_2H_5$ | 1 | 4-$CH_3$ | 144-144.5 |
| 22 | 1 | 5-$C_2H_5$ | 1 | 4-$OCH_3$ | 100-102 |
| 23 | 1 | 5-$C_2H_5$ | 1 | 4-$OC_2H_5$ | 120-122 |
| 24 | 1 | 5-$C_2H_5$ | 1 | 4-O*n*-$C_3H_7$ | 110-115 |
| 25 | 1 | 5-$CH_3$ | 1 | 4-$OCH_3$ | 135-139 |
| 26 | 1 | 5-$CH_3$ | 1 | 5(6)-$CH_3$ | 144-145 |

| Cpd. No | n | Y | m | $Y^1$ | m.p./ C |
|---|---|---|---|---|---|
| 27 | 1 | 5-$CH_3$ | 1 | 4-$CH_3$ | 134-137 |
| 28 | 1 | 5-$CH_3$ | 1 | 5-Cl | 213-218 |
| 29 | 1 | 5-$CH_3$ | 1 | 4-O*n*-$C_3H_7$ | 110-114 |
| 30 | 1 | 5-$CH_3$ | 1 | 4-$OC_2H_5$ | 119-122 |
| 31 | 1 | 5-$CH_3$ | 1 | 4-O*i*-$C_3H_7$ | 125-128 |
| 32 | 1 | 5-$C_2H_5$ | 1 | 4-O*i*-$C_3H_7$ | 105-108 |

**EXAMPLE 3**

Preparation of isopropylammonium 2-[1-(N,N-dimethylsulphamoyl)-5-chloro-benzimidazol-2-yl]-6-methylpyridine-3-carboxylate, compound 33.

Isopropylamine (0.21 g) was added to a stirred suspension of 2-[1-(N,N-dimethyl-sulphamoyl)-5-chloro-benzimidazol-2-yl]-6-methylpyridine-3-carboxylic acid (0.99 g) in acetone (10 ml) at room temperature. After a few seconds a clear solution was obtained this was followed 5 minutes later by the formation of a white precipitate. The mixture was stirred for a further 6.25 hours The solvent was evaporated and the residue dried to give the title compound (1.18 g) as a pale yellow solid, m.p. 140 °C (Yield: 100%).

By proceeding in a similar manner the salts of the formula below were obtained form the corresponding acid:

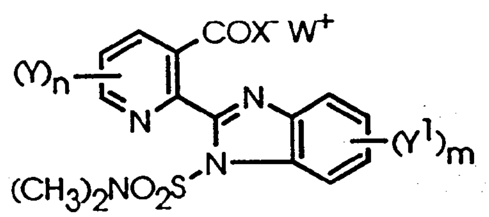

| Cpd. No. | n | Y | m | $Y^1$ | X | W | m.p. /$^oC$ |
|---|---|---|---|---|---|---|---|
| 34 | 1 | 5-$CH_3$ | 1 | 4-$CH_3$ | O | K | 163-165 |
| 35 | 1 | 5-$CH_3$ | 1 | 4-$CH_3$ | O | iPr-$NH_3$ | 157-159 |
| 36 | 1 | 5-$CH_3$ | 1 | 5-Cl | O | K | 234-236 |
| 37 | 1 | 5-$CH_3$ | 1 | 5-Cl | O | iPr-$NH_3$ | 98-100 |

## REFERENCE EXAMPLE 1

Preparation of 2-methylpyrido[2',3',3,4]pyrrolo[1,2-a]-7-methoxybenzimidazol-5-one

Finely powdered 6-methylpyridine-2,3-dicarboxylic acid anhydride (17.5g) and 2,3-diaminoanisole (14.8g) were intimately mixed and then heated to 165°C. After 1 hour a vacuum was applied to remove the water which was present, and heating continued for 15 minutes. Acetic anhydride (60 ml) was added to the cooled reaction mixture and the resulting mixture was refluxed for 2 hours. After cooling diethyl ether was added and the brown precipitate thus obtained was filtered and dried to give the title compound (10.5 g) as a brown solid, m.p. 260-261°C (Yield: 37%).

By proceeding in a similar manner, the compounds of the general formula described in the following table were obtained:

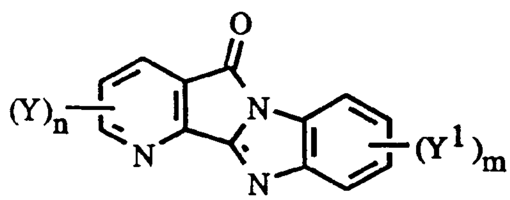

| n | Y | m | $Y^1$ | m.p./ °C |
|---|---|---|---|---|
| 1 | 2-$CH_3$ | 1 | 8(9)-F | 253-255 |
| 1 | 2-$CH_3$ | 1 | 7-$OC_2H_5$ | 187-189 |
| 1 | 2-$CH_3$ | 1 | 7,8-di$CH_3$ | 247-250 |
| 1 | 2-$CH_3$ | 1 | 8(9)-Cl | >250 |
| 1 | 2-$CH_3$ | 1 | 5(6)-$CH_3$ | 295-300 |
| 1 | 2-$CH_3$ | 1 | 7-$CH_3$ | 250-252.5 |
| 1 | 2-$CH_3$ | 1 | 7-O *n*$C_3H_7$ | 170-177 |
| 1 | 2-$CH_3$ | 1 | 7-O*i*-$C_3H_7$ | 138-140 |
| 1 | 2-$CH_3$ | 1 | 7-Cl | not isolated |
| 1 | 3-$C_2H_5$ | 1 | 8(9)-Cl | nmr (m) |
| 1 | 3-$C_2H_5$ | 1 | 7-$CH_3$ | 170-174 |
| 1 | 3-$C_2H_5$ | 1 | 7-$OCH_3$ | not isolated |
| 1 | 3-$C_2H_5$ | 1 | 7-$OC_2H_5$ | 155-160 |
| 1 | 3-$C_2H_5$ | 1 | 7-O*n*-$C_3H_7$ | 161-163 |
| 1 | 3-$CH_3$ | 1 | 7-$OCH_3$ | 226-229 |
| 1 | 3-$CH_3$ | 1 | 8(9)-$CH_3$ | 217-220 |
| 1 | 3-$CH_3$ | 1 | 7-$CH_3$ | 226-229 |
| 1 | 3-$CH_3$ | 1 | 8(9)-Cl | 225-228 |
| 1 | 3-$CH_3$ | 1 | 7-O*n*-$C_3H_7$ | 214-217 |
| 1 | 3-$CH_3$ | 1 | 7-$OC_2H_5$ | 230-235 |
| 1 | 3-$CH_3$ | 1 | 7-O*i*-$C_3H_7$ | 170-175 |
| 1 | 3-$C_2H_5$ | 1 | 7-O*n*-$C_3H_7$ | 161-163 |

$H^1$ NMR

(m) ($CDCl_3$) d= 1.4 (3H, t), 2.75 (2H, q), 7.25 (1H, m), 7.6 (1H, m) 7.7 (1H, m), 7.85 (1H, s), 8.6 (1H, s) ppm.

## REFERENCE EXAMPLE 2

Preparation of methyl 2-(4-methoxy-1*H*-benzimidazol-2-yl)-6-methylpyridine-3-carboxylate.

Triethylamine (3ml) was added to a stirred suspension of 2-methylpyrido[2′,3′,3,4]pyrrolo[1,2-a]-7-methoxybenzimidazol-5-one (5g) in dichloromethane (100ml) at room temperature. After 0.5 hour methanol (1ml) was added to the resulting brown solution which was stirred at room temperature for 14 hours, then washed with water, dried over magnesium sulphate and the solvent evaporated. The title compound (4.6g) was obtained in the form of a yellow solid, m.p. 62-64°C (Yield: 78%).

By proceeding in a similar manner the following compounds of the general formula shown below were obtained from the appropriate pyrido[2′,3′:3,4]pyrrolo[1,2-a]benzimidazol-5-one and alcohol:

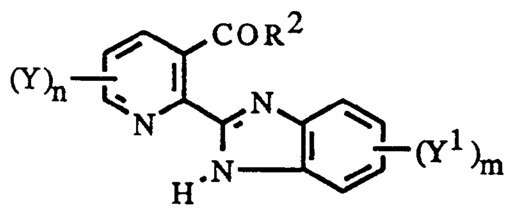

| n | Y | m | Y1 | $R^2$ | m.p./ C |
|---|---|---|---|---|---|
| 1 | 6-$CH_3$ | 1 | 4-$OCH_3$ | $OCH_2C{\equiv}CH$ | 138-139.5 |
| 1 | 6-$CH_3$ | 1 | 5(6)-F | $OCH_3$ | 145-146.5 |
| 1 | 6-$CH_3$ | 1 | 4-$OC_2H_5$ | $OCH_3$ | 159-161 |
| 1 | 6-$CH_3$ | 1 | 4-$OC_2H_5$ | $OCH_2C{\equiv}CH$ | 160.5-161.5 |
| 1 | 6-$CH_3$ | 1 | 4,5-di$CH_3$ | $OCH_3$ | 202-204 |
| 1 | 6-$CH_3$ | 1 | 5(6)-Cl | $OCH_3$ | 145-146.5 |
| 1 | 6-$CH_3$ | 1 | 5(6)-Cl | $OCH_2C{\equiv}CH$ | 175-177 |
| 1 | 6-$CH_3$ | 1 | 5(6)-$CH_3$ | $OCH_3$ | 154.4-155.6 |
| 1 | 6-$CH_3$ | 1 | 5(6)-$CH_3$ | $OCH_2C{\equiv}CH$ | 148.5-151 |

| n | Y | m | $Y^1$ | $R^2$ | m.p./ C |
|---|---|---|---|---|---|
| 1 | 6-$CH_3$ | 1 | 4-$CH_3$ | $OCH_3$ | 127-128 |
| 1 | 6-$CH_3$ | 1 | 4-O*n*-$C_3H_7$ | $OCH_3$ | 125-131 |
| 1 | 6-$CH_3$ | 1 | 4-O*i*-$C_3H_7$ | $OCH_3$ | glass nmr (i) |
| 1 | 6-$CH_3$ | 1 | 4-Cl | $OCH_3$ | 182-183 |
| 1 | 5-$C_2H_5$ | 1 | 5(6)-Cl | $OCH_3$ | 82-84 |
| 1 | 5-$C_2H_5$ | 1 | 4-$CH_3$ | $OCH_3$ | gum, nmr (j) |
| 1 | 5-$C_2H_5$ | 1 | 4-$OCH_3$ | $OCH_2C{\equiv}CH$ | 107-109 |
| 1 | 5-$C_2H_5$ | 1 | 4-$OC_2H_5$ | $OCH_3$ | 60-62 |
| 1 | 5-$C_2H_5$ | 1 | 4-O*n*-$C_3H_7$ | $OCH_3$ | 55-57 |
| 1 | 5-$CH_3$ | 1 | 4-$OCH_3$ | $OCH_2C{\equiv}CH$ | 169-170 |
| 1 | 5-$CH_3$ | 1 | 5(6)-$CH_3$ | $OCH_2C{\equiv}CH$ | 161-163 |
| 1 | 5-$CH_3$ | 1 | 4-$CH_3$ | $OCH_2C{\equiv}CH$ | 177-178.5 |
| 1 | 5-$CH_3$ | 1 | 5(6)-Cl | $OCH_2C{\equiv}CH$ | 176.3-177.3 |
| 1 | 5-$CH_3$ | 1 | 4-O*n*-$C_3H_7$ | $OCH_3$ | gum, nmr (k) |
| 1 | 5-$CH_3$ | 1 | 4-$OC_2H_5$ | $OCH_3$ | 47-49 |
| 1 | 5-$CH_3$ | 1 | 4-O*i*-$C_3H_7$ | $OCH_3$ | gum, nmr (l) |
| 1 | 5-$C_2H_5$ | 1 | 4-O *i*-$C_3H_7$ | $OCH_3$ | 48.5-53.5 |

$H^1$ NMR.

(i) (DMSO-$d_6$) d= 1.30 (6H, d), 2.65 (3H, s), 3.85 (3H, s), 5.15 (1H, m), 6.75 (1H,m), 7.10 (2H,m), 7.45 (1H, d), 7.90 (1H, d), 12.90 (1H, s) ppm.

(j) ($CDCl_3$) d= 1.30 (3H, t), 2.60 (3H, s), 2.75 (2H, q), 4.00 (3H, s), 7.05 (1H, d), 7.15 (1H, t), 7.40, (1H, d), 7.70 (1H, d), 8.55 (1H, d) ppm.

(k) ($CDCl_3$) d= 1.10 (3H, t), 1.85, (2H, sx), 2.4 (3H, s), 4.05 (3H, s), 4.15 (2H, t), 6.70 (1H, d), 7.15 (1H, t), 7.25 (1H, d), 7.7 (1H, d), 8.55 (1H, d)

(I) ($CDCl_3$) d= 1.33 (6H,d), 2.35 (3H, s), 3.95 (3H, s), 4.80 (1H, m),

6.65 (1H, d), 7.10 (1H,t), 7.18 (1H, d), 7.63 (1H, d), 8.45 (1H, d) ppm.

## REFERENCE EXAMPLE 3

Preparation of N-(4-methyl-nitrophenyl)maleamide.

A mixture of 4-methyl-2-nitroaniline (10 g) and maleic anhydride (6.45 g) in dry dichloromethane (50 ml) was stirred at room temperature for 24 hours. The original orange suspension disappeared and was replaced by a bright yellow solid. This was filtered off and washed with cold dichloromethane to give the title compound (12.5 g) as a pale yellow solid, m.p. 127-129 °C (Yield: 76%).

By proceeding in a similar manner the following compound was prepared: N-(4-methoxy-2-nitrophenyl)maleamide, m.p. 144-146 °C.

## REFERENCE EXAMPLE 4

Preparation of N-(4-methyl-2-nitrophenyl)maleimide.

A stirred mixture of N-(4-methyl-2-nitrophenyl)maleamide (12 g) and dry sodium acetate (5.65 g) in acetic anhydride (90 ml) was heated at 100 °C for 3 hours. After cooling the mixture was poured onto ice/water (100 ml) and stirred for a further 3 hours. The resulting precipitate was filtered and dried to give the title compound (8 g) as a yellow crystalline solid, m.p. 104-106 °C (Yield: 72%).

By proceeding in a similar manner the following compound was prepared: N-(4-methoxy-2-nitrophenyl)maleimide, m.p. 110-112 °C.

## REFERENCE EXAMPLE 5

Preparation of N-(4-methyl-2-nitrophenyl)-5-ethyl-1,4-dihydropyridine-2,3-dicarboximide.

A stirred mixture of 1-(N,N-dimethylamino-2-ethyl-1-aza-1,3-butadiene (30.24 g) and N-(4-methyl-2-nitrophenyl)maleimide (60 g) in dry acetonitrile (500 ml) was heated at reflux for 3 hours. After cooling the solvent was removed to leave a deep red semi-solid which was triturated with ethyl acetate, filtered and dried to give the title compound (30 g) as a deep red solid, m.p. 202-203.6 °C (Yield: 42%).

By proceeding in a similar manner the following compound was prepared: N-(4-methoxy-2-nitrophenyl)5-ethyl-1,4-dihydropyridine-2,3-dicarboximide, m.p. 194.5-195.5 °C.

## REFERENCE EXAMPLE 6

Preparation of N-(4-methyl-2-nitrophenyl)-5-ethylpyridine-2,3-dicarboximide.

A stirred solution of N-(4-methyl-2-nitrophenyl)-5-ethyl-1,4-dihydropyridine-2,3-dicarboximide (25 g) in acetic acid (200 ml) was heated at 60 °C. Heating was ceased and manganese(II)oxide (7.7 g) was added giving an exotherm with the temperature reaching 80 °C. After complete addition the resulting black suspension was stirred until room temperature was attained. The reaction mixture was filtered through and the filtrate concentrated *in vacuo* to give a semi-solid which was recrystallised from ethyl acetate to give the title compound (12 g) as a light biege solid, m.p. 172-173.5 °C (Yield: 51%).

By proceeding in a similar manner the following compound was prepared: N-(4-methoxy-2-nitrophenyl)-5-ethylpyridine-2,3-dicarboximide.

## REFERENCE EXAMPLE 7

Preparation of N-(2-amino-4-methylphenyl)-5-ethylpyridine-2,3-dicarboximide.

A mixture of N-(4-methyl-2-nitrophenyl)-5-ethylpyridine-2,3-dicarboximide (10 g) and 5% palladium on charcoal catalyst (0.5 g) in dioxan (100 ml) was stirred at 60 °C under an atmosphere of hydrogen. The reaction

mixture was cooled to room temperature and the catalyst removed by filtration. Evaporatiuon of the filtrate gave a glassy solid which was purified by MPLC eluting with a mixture of hexane and ethyl acetate to give the title compound (3.6 g) as a yellow solid, (Yield: 43%), $H^1$ NMR ($CDCl_3$) ; d= 1.3 (3H, t), 2.25 (3H,s), 2.8 (2H, q), 3.6 (2H, bs), 6.7 (2H, m), 6.9 (1H, d), 8.0 (1H, d), 8.8 (1H, d) ppm.

By proceeding in a similar manner the following compound was prepared: N-(2-amino-4-methoxyphenyl)-5-ethylpyridine-2,3-dicarboximide, $H^1$ NMR (DMSO-$d_6$) d= 1.25 (3H, t), 2.7 (2H, q), 3.7 (3H, s), 5.4 (2H, s), 6.2 (1H, dd), 6.3 (1H, d), 7.0 (1H, d), 8.2 (1H, d), 8.9 (1H, d) ppm.

**REFERENCE EXAMPLE 8** Preparation of ethoxyethyl 2-(5-methyl-1*H*-benzimidazol-2-yl)-5-ethylpyridine-3-carboxylate.

A stirred solution of N-(2-amino-4-methylphenyl)-5-ethylpyridine-2,3-dicarboximide (2.5 g) in ethoxyethanol (35 ml) was heated at reflux for 24 hours. The solvent was removed and the residue purified by MPLC eluting with ethyl acetate/hexane to give the title compound (1.1 g) as an orange solid, m.p. 113.5-115 °C.

By proceeding in a similar manner the following compound was prepared: ethoxyethyl 2-(5-methoxy-1*H*-benzimidazol-2-yl)-5-ethylpyridine-3-carboxylate, $H^1$ NMR ($CDCl_3$); d= 1.0 (3H, t), 1.2 (3H,t),2.65 (2H, q), 3.9 (2H, q), 4.55 (2H, q), 6.8 (1H, bd), 7.5 (1H, bs), 7.7 (1H, d), 8.45 (1H, d) ppm.

**REFERENCE EXAMPLE 9**

Preparation of 2-methoxy-6-nitroaniline.

Anhydrous potassium carbonate (35.8g) was added portionwise to a stirred solution of 2-amino-3-nitrophenol# (40g) in acetone (56ml). Methyl iodide (37.7g) was added and the mixture heated at reflux overnight under an inert atmosphere. After cooling the reaction mixture was poured into water (200 ml) and extracted with ethyl acetate, The organic layer was washed with aqueous sodium hydroxide solution (2 x 100ml) and water. After drying over magnesium sulphate and evaporation of solvent the residue was purified by dry column chromatography on silica gel eluting with a 4:1 mixture of dichloromethane and ethyl acetate to give the title compound as a brown solid (40.3g), m.p. 75-77°C (Yield: 92%).

# As described by E. Fourneau and J. Trefouel, Bull. Soc. Chim. France, 1927, 41, 448.

**REFERENCE EXAMPLE 10**

Preparation of 2,3-diaminoanisole.

A mixture of 2-methoxy-6-nitroaniline (40.3g) and palladium black (0.45g) in ethanol (500ml) was vigorously stirred at 45°C under an atmosphere of hydrogen. After the uptake of hydrogen ceased the catalyst was removed, washed with ethanol and the combined organic solutions evaporated to give the title compound as a brown oil (33.5g,100%) with the $H^1$ N.M.R of the oil ($CDCl_3$) giving peaks at d= 3.5 (4H, br, s), 3.85 (3H, s), 6.3 (1H, dd), 6.4 (1H, dd) and 6.7 (1H, t) ppm.

**EXAMPLE 4**

Preparation of 2-[1-(N,N-dimethylsulphamoyl)-4-methoxybenzimidazol-2-yl]quinoline-3-carboxylic acid, compound 38.

A mixture of methyl 2-[1-(N,N-dimethylsulphamoyl)-4-methoxybenzimidazol-2-yl]-quinoline-3-carboxylate (5.25 g) and lithium hydroxide (1.5 g) in water (15 ml) and methanol (50 ml) was stirred at room temperature for 12 hours. The mixture was filtered, evaporated and the residue dissolved in water and washed with dichloromethane. The aqueous solution was then acidified with 1 M hydrochloric acid to give a white precipitate which was filtered and dried to give the title compound (1.6 g) as a white solid, m.p. 192-194 °C (Yield: 36%).

By proceeding in a similar manner the compounds of the formula shown below were prepared.

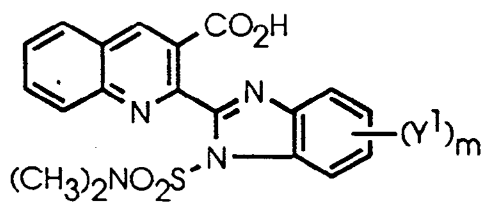

| Compound No. | m | Y1 | m.p. / °C |
|---|---|---|---|
| 39 | 1 | 4-$OC_2H_5$ | 195-196 |
| 40 | 2 | 4,5-di$CH_3$ | 242-244 |
| 41 | 1 | 5(6)-$CH_3$ | 231-234 |
| 42 | 1 | 4-Oi$C_3H_7$ | 175-176 |
| 43 | 1 | 4-$CH_3$ | 204-230 |
| 44 | 1 | 5-Cl | 273 |

## EXAMPLE 5

Preparation of methyl 2-[1-(N,N-dimethylsulphamoyl)-4-methoxybenzimidazol-2-yl]-quinoline-3-carboxylate, compound 48.

A mixture of anhydrous potassium carbonate and methyl 2-(4-methoxy-1H-benzimidazol-2-yl)quinoline-3-carboxylate (11 g) in acetonitrile (130 ml) was heated at reflux for 2 hours. The resulting yellow suspension was cooled and dimethylsulphamoyl chloride (14.2 g) added. The mixture was then heated at reflux for 6 hours. After cooling the solvent was evaporated and the residue partitioned between water and dichloromethane. The organic phase was washed with water, dried and evaporated to give a yellow solid which was recrystallised from ethyl acetate to give the title compound (6.25 g) as a cream solid, m.p. 195-197 °C (Yield : 58%).

By proceeding in a similar manner the following compounds were obtained:

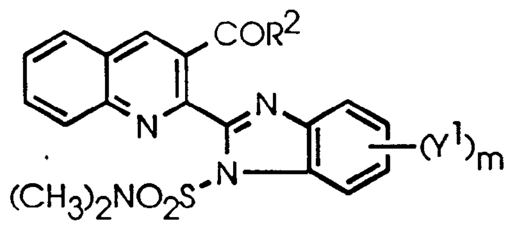

| Compound No | m | $Y^1$ | $R^2$ | m.p. / °C |
|---|---|---|---|---|
| 49 | 1 | 4-$OC_2H_5$ | $OCH_3$ | 177-178 |
| - | 2 | 4,5-di$CH_3$ | $OCH_3$ | 225-227 |
| 45 | 1 | 5(6)-$CH_3$ | $OCH_2C{\equiv}CH$ | 173.8-177.3 |
| - | 1 | 4-O*i*$C_3H_7$ | $OCH_3$ | NMR (n) |
| - | 1 | 5(6)-Cl | $OCH_3$ | NMR (o) |
| - | 1 | 4-$CH_3$ | $OCH_3$ | 210-212 |

$H^1$ NMR.

(n) (DMSO-$d_6$) d= 1.30 (6H, t), 2.85 (6H, s), 3.75 (3H, s), 4.95 (1H, m), 6.95 (1H, d), 7.35 (1H, t), 7.45 (1H, d), 7.85 (1H, t), 8.00 (1H, t), 8.10 (1H, d), 8.45 (1H, d), 9.20 (1H,s) ppm.

(o) (DMSO-$d_6$) d= 2.85 (6H, s), 3.75 (3H, s), 7.55 (1H, t), 7.90 (3H, m), 8.05 (1H,t), 8.15 (1H, d), 8.35 (1H, d), 9.2 (1H, s) ppm.

## REFERENCE EXAMPLE 11

Preparation of methyl 2-(4-methoxy-1H-benzimidazol-2-yl)quinoline-3-carboxylate.

Triethylamine (5.2 ml) was added to a stirred suspension of quinolo[2′,3′;3,4]pyrrolo[1,2-a]-9-methoxybenzimidazol-7-one (10.8 g) in dichloromethane at room temperature. After 14 hours the solution was added to water. The organic phase was washed with water, dried and evaporated to give a yellow gum which was purified by dry column chromatography eluting with dichloromethane to give the title compound (11g) as a yellow solid, m.p. 84-86 °C (Yield: 92%).

By proceeding in a similar manner the following compounds were prepared from the appropriate quinolo[2′,3′:3,4]pyrrolo[1,2-a]benzimidazol-7-one and alcohol.

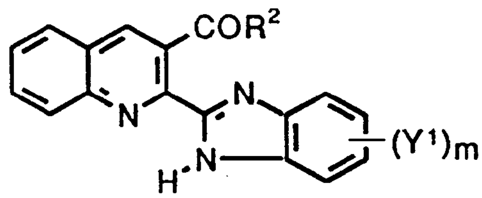

| m | $Y^1$ | $R^2$ | m.p. /°C |
|---|---|---|---|
| 1 | 4-$OC_2H_5$ | $OCH_3$ | 71-78 |
| 2 | 4,5-di$CH_3$ | $OCH_3$ | 225-227 |
| 1 | 5(6)-$CH_3$ | $OCH_2C{\equiv}CH$ | 173.8-177.3 |
| 1 | 4-Oi$C_3H_7$ | $OCH_3$ | nmr (p) |
| 1 | 4-$CH_3$ | $OCH_3$ | nmr (q) |
| 1 | 5(6)-Cl | $OCH_3$ | 173-174 |

$H^1$ NMR.

(p) (DMSO-$d_6$) d= 1.35 (6h, d), 3.95 (3H, s), 5.15 (1H, m), 6.70 (1H, m), 7.20 (2H, m), 7.75 (1H, s), 7.95 (1H, t), 8.10 (1H, d), 8.20 (1H, d), 8.70 (1H, s), 13.2 (1H, s) ppm.

(q) (DMSO-$d_6$) d= 2.60 (3H, s), 4.95 (3H, s), 7.05 (1H, d), 7.20 (1H, t), 7.45 (1H, d), 7.75 (1H, m), 7.95 (1H, m), 8.15 (1H, d), 8.20 (1H, d), 8.70 (1H, s), 13.20 (1H, s).

**REFERENCE EXAMPLE 12.**

Preparation of quinolo[2',3':3,4]pyrrolo[1,2-a]-9-methoxybenzimidazol-7-one.

A mixture of quinoline-2,3-dicarboxylic acid anhydride (11.6 g) and 2,3-diaminoanisole (8.0 g) in acetic acid (45 ml) was stirred and heated at reflux for 12 hours. Acetic anhydride (23 ml) was added to the cooled reaction mixture and the mixture was heated at reflux for 3 hours. The mixture was allowed to cool to room temperature and the resulting precipitate was filtered, washed with ether and dried to give the title compound (10.8 g) as a yellow solid, m.p. 286-288 °C (Yield: 61.5%).

By proceeding in a similar manner the following compounds of general formula shown below were obtained from the appropriate benzene-1,2-diamine:

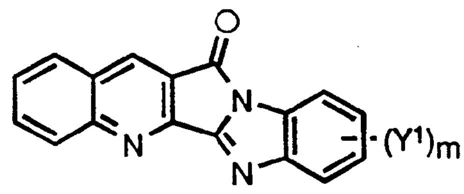

| m | $Y^1$ | m.p. /°C |
|---|---|---|
| 1 | 9-$OC_2H_5$ | 275-280 |
| 2 | 9,10-$(CH_3)_2$ | 275-278 |
| 1 | 10(11)-$CH_3$ | 235-242 |
| 1 | 9-$OiC_3H_7$ | 252-253 |
| 1 | 9-$CH_3$ | 260-261 |
| 1 | 10(11)-Cl | 161-162 |

According to a feature of the present invention, there is provided a method for controlling the growth of weeds (i.e. undesired vegetation) at a locus which comprises applying to the locus a herbicidally effective amount of at least one benzimidazolyl derivative of general formula (I) or an agriculturally acceptable salt thereof. For this purpose, the benzimidazolyl derivatives are normally used in the form of herbicidal compositions (i.e. in association with compatible diluents or carriers and/or surface active agents suitable for use in herbicidal compositions), for example as hereinafter described.

The compounds of general formula (I) show herbicidal activity against dicotyledonous (i.e. broad-leafed) and monocotyledonous (e.g. grass) weeds by pre- and/or post-emergence application.

By the term "pre-emergence application" is meant application to the soil in which the weed seeds or seedlings are present before emergence of the weeds above the surface of the soil. By the term "post-emergence application" is meant application to the aerial or exposed portions of the weeds which have emerged above the surface of the soil. For example, the compounds of general formula (I) may be used to control the growth of:

broad-leafed weeds, for example, Abutilon theophrasti, Amaranthus retroflexus, Bidens pilosa, Chenopodium album, Galium aparine, Ipomoea spp. e.g. Ipomoea purpurea, Sesbania exaltata, Sinapis arvensis, Solanum nigrum and Xanthium strumarium, and

grass weeds, for example Alopecurus myosuroides, Avena fatua, Digitaria sanguinalis, Echinochloa crus-galli, Eleusine indica and Setaria spp, e.g. Setaria faberii or Setaria viridis, and

sedges, for example, Cyperus esculentus.

The amounts of compounds of general formula (I) applied vary with the nature of the weeds, the compositions used, the time of application, the climatic and edaphic conditions and (when used to control the growth of weeds in cropgrowing areas) the nature of the crops. When applied to a crop-growing area, the rate of application should be sufficient to control the growth of weeds without causing substantial permanent damage to the crop. In general, taking these factors into account, application rates between 0.01kg and 5kg of active material per hectare give good results. However, it is to be understood that higher or lower application rates may be used, depending upon the particular problem of weed control encountered.

The compounds of general formula (I) may be used to control selectively the growth of weeds, for example to control the growth of those species hereinbefore mentioned, by pre- or post-emergence application in a directional or non-directional fashion, e.g. by directional or non-directional spraying, to a locus of weed infestation which is an area used, or to be used, for growing crops, for example cereals, e.g. wheat, barley, oats, maize and rice, soya beans, field and dwarf beans, peas, lucerne, cotton, peanuts, flax, onions, carrots, cabbage, oilseed rape, sunflower, sugar beet, and permanent or sown grassland before or after sowing of the crop or before or after emergence of the crop. For the selective control of weeds at a locus of weed infestation which is an area used, or to be used, for growing of crops, e.g. the crops hereinbefore mentioned, application rates between 0.01kg and 4.0kg, and preferably between 0.01kg and 2.0kg, of active material per hectare are particularly suitable.

The compounds of general formula (I) may also be used to control the growth of weeds, especially those indicated above, by pre- or post-emergence application in established orchards and other tree-growing areas, for example forests, woods and parks, and plantations, e.g. sugar cane, oil palm and rubber plantations. For this purpose they may be applied in a directional or non-directional fashion (e.g. by directional or non-directional spraying) to the weeds or to the soil in which they are expected to appear, before or after planting of the tees or plantations at application rates between 0.25kg and 5.0kg, and preferably between 0.5kg and 4.0kg of active material per hectare.

The compounds of general formula (I) may also be used to control the growth of weeds, especially those indicated above, at loci which are not crop-growing areas but in which the control of weeds in nevertheless desirable.

Examples of such non-crop-growing areas include airfields, industrial sites, railways, roadside verges, the verges of rivers, irrigation and other waterways, scrublands and fallow or uncultivated land, in particular where it is desired to control the growth of weeds in order to reduce fire risks. When used for such purposes in which a total herbicidal effect is frequently desired, the active compounds are normally applied at dosage rates higher than those used in crop-growing areas as hereinbefore described. The precise dosage will depend upon the nature of the vegetation treated and the effect sought.

Pre- or post-emergence application, and preferably pre-emergence application, in a directional or non-directional fashion (e.g. by directional or non-directional spraying) at application rates between 1.0kg and 20.0kg, and preferably between 5.0 and 10.0kg, of active material per hectare are particularly suitable for this purpose.

When used to control the growth of weeds by pre-emergence application, the compounds of general formula (I) may be incorporated into the soil in which the weeds are expected to emerge. It will be appreciated that when the compounds of general formula (I) are used to control the growth of weeds by post-emergence application, i.e. by application to the aerial or exposed portions of emerged weeds, the compounds of general formula (I) will also normally come into contact with the soil and may also then exercise a pre-emergence control on later-germinating weeds in the soil.

Where especially prolonged weed control is required, the application of the compounds of general formula (I) may be repeated if required.

According to a further feature of the present invention, there are provided compositions suitable for herbicidal use comprising one or more of the benzimidazolyl derivatives of general formula I or an agriculturally acceptable salt thereof, in association with, and preferably homogeneously dispersed in, one or more compatible agriculturally- acceptable diluents or carriers and/or surface active agents [i.e. diluents or carriers and/or surface active agents of the type generally accepted in the art as being suitable for use in herbicidal compositions and which are compatible with compounds of general formula (I)]. The term "homogeneously dispersed" is used to include compositions in which the compounds of general formula (I) are dissolved in other components. The term "herbicidal composition" is used in a broad sense to include not only compositions which are ready for use as herbicides by also concentrates which must be diluted before use. Preferably, the compositions contain from 0.05 to 90% by weight of one or more compounds of general formula (I).

The herbicidal compositions may contain both a diluent or carrier and surface-active (e.g. wetting, dispersing, or emulsifying) agent. Surface-active agents which may be present in herbicidal compositions of the present invention may be of the ionic or non-ionic types, for example sulphoricinoleates, quaternary ammonium derivatives, products based on condensates of ethylene oxide with alkyl and polyaryl phenols, e.g. nonyl- or octylphenols, or carboxylic acid esters of anhydrosorbitols which have been rendered soluble by etherification of the free hydroxy groups by condensation with ethylene oxide, alkali and alkaline earth metal salts of sulphuric acid esters and sulphonic acids such as dinonyl- and dioctyl-sodium sulphonosuccinates and alkali and alkaline earth metal salts of high molecular weight sulphonic acid derivatives such as sodium and calcium lignosulphonates and sodium and calcium alkylbenzene sulphonates.

Suitably, the herbicidal compositions according to the present invention may comprise up to 10% by weight, e.g. from 0.05% to 10% by weight, of surface-active agent but, if desired, herbicidal compositions according to the present invention may comprise higher proportions of surface-active agent, for example up to 15% by weight in liquid emulsifiable suspension concentrates and up to 25% by weight in liquid water soluble concentrates.

Examples of suitable solid diluents or carriers are aluminium silicate, talc, calcined magnesia, kieselguhr, tricalcium phosphate, powdered cork, adsorbent carbon black and clays such as kaolin and bentonite. The solid compositions (which may take the form of dusts, granules or wettable powders) are preferably prepared by grinding the compounds of general formula (I) with solid diluents or by impregnating the solid diluents or carriers with solutions of the compounds of general formula (I) in volatile solvents, evaporating the solvents and, if necessary, grinding the products so as to obtain powders. Granular formulations may be prepared by absorbing the compounds of general formula (I) (dissolved in suitable solvents, which may, if desired, be volatile) onto

the solid diluents or carriers in granular form and, if desired, evaporating the solvents, or by granulating compositions in powders form obtained as described above. Solid herbicidal compositions, particularly wettable powders and granules, may contain wetting or dispersing agents (for example of the types described above), which may also, when solid, serve as diluents or carriers.

Liquid compositions according to the invention may take the form of aqueous, organic or aqueous-organic solutions, suspensions and emulsions which may incorporate a surface-active agent. Suitable liquid diluents for incorporation in the liquid compositions include water, glycols, tetrahydrofurfuryl alcohol, acetophenone, cyclohexanone, isophorone, toluene, xylene, mineral, animal and vegetable oils and light aromatic and naphthenic fractions of petroleum (and mixtures of these diluents). Surface-active agents, which may be present in the liquid compositions, may be ionic or non-ionic (for example of the types described above) and may, when liquid, also serve as diluents or carriers.

Powders, dispersible granules and liquid compositions in the form of concentrates may be diluted with water or other suitable diluents, for example mineral or vegetable oils, particularly in the case of liquid concentrates in which the diluent or carrier is an oil, to give compositions ready for use.

When desired, liquid compositions of the compound of general formula (I) may be used in the form of self-emulsifying concentrates containing the active substances dissolved in the emulsifying agents or in solvents containing emulsifying agents compatible with the active substances, the simple addition of water to such concentrates producing compositions ready for use.

Liquid concentrates in which the diluent or carrier is an oil may be used without further dilution using the electrostatic spray technique.

Herbicidal compositions according to the present invention may also contain, if desired, conventional adjuvants such as adhesives, protective colloids, thickeners, penetrating agents, stabilisers, sequestering agents, anti-caking agents, colouring agents and corrosion inhibitors. These adjuvants may also serve as carriers or diluents.

Unless otherwise specified, the following percentages are by weight. Preferred herbicidal compositions according to the present invention are aqueous suspension concentrates which comprise from 10 to 70% of one or more compounds of general formula (I), from 2 to 10% of surface-active agent, from 0.1 to 5% of thickener and from 15 to 87.9% of water;

wettable powders which comprise from 10 to 90% of one or more compounds of general formula (I), from 2 to 10% of surface-active agent and from 8 to 88% of solid diluent or carrier;

water soluble or water dispersible powders which comprise from 10 to 90% of one or more compounds of general formula (I), from 2 to 40% of sodium carbonate and from 0 to 88% of solid diluent;

liquid water soluble concentrates which comprise from 5 to 50%, e.g. 10 to 30%, of one or more compounds of general formula (I), from 5 to 25% of surface-active agent and from 25 to 90%, e.g. 45 to 85%, of water miscible solvent, e.g. dimethylformamide, or a mixture of water-miscible solvent and water;

liquid emulsifiable suspension concentrates which comprise from 10 to 70% of one or more compounds of general formula (I), from 5 to 15% of surface-active agent, from 0.1 to 5% of thickener and from 10 to 84.9% of organic solvent;

granules which comprise from 1 to 90%, e.g. 2 to 10% of one or more compounds of general formula (I), from 0.5 to 7%, e.g. 0.5 to 2%, of surface-active agent and from 3 to 98.5%, e.g. 88 to 97.5%, of granular carrier and

emulsifiable concentrates which comprise 0.05 to 90%, and preferably from 1 to 60% of one or more compounds of general formula (I), from 0.01 to 10%, and preferably from 1 to 10%, of surface-active agent and from 9.99 to 99.94%, and preferably from 39 to 98.99%, of organic solvent.

Herbicidal compositions according to the present invention may also comprise the compounds of general formula (I) in association with, and preferably homogeneously dispersed in, one or more other pesticidally active compounds and, if desired, one or more compatible presticidally acceptable diluents or carriers, surface-active agents and conventional adjuvants as hereinbefore described. Examples of other presticidally active compounds which may be included in, or used in conjunction with, the herbicidal compositions of the present invention include herbicides, for example to increase the range of weed species controlled for example alachlor [2-chloro-2,6′-diethyl-N-(methoxy-methyl)-acetanilide], atrazine [2-chloro-4-ethylamino-6-isopropylamino-1,3,5-triazine], bromoxynil [3,5-dibromo-4-hydroxybenzonitrile], chlortoluron [N′-(3-chloro-4-methylphenyl)-N,N-dimethylurea], cyanazine [2-chloro-4-(1-cyano-1-methylethylamino)-6-ethylamino-1,3,5-triazine], 2,4-D[2,4-dichlorophenoxy-acetic acid], dicamba [3,6-dichloro-2-methoxybenzoic acid], difenzoquat [1,2-dimethyl-3,5-diphenyl-pyrazolium salts], flampropmethyl [methyl N-2-(N-benzoyl-3-chloro-4-fluoroanilino)-propionate], fluometuron [N′-(3-trifluoro- methylphenyl)-N,N-dimethylurea], isoproturon [N′-(4-isopropyl-phenyl)-N,N-dimethylurea], nicosulfuron [2-(4′,6′dimethoxypyrimidin-2′-ylcarbamoylsulfamoyl)-N,N-dimethylnicotinamide], insecticides, e.g. synthetic pyrethroids, e.g. permethrin and cypermethrin, and fungicides, e.g. carbamates, e.g. methyl N-(1-butyl-carbamoyl-benzimidazol-2-yl)carbamate, and triazoles e.g. 1-(4-chlorophenoxy)-3,3-dimethyl-1-(1,2,4-triazol-1-yl)-butan-2-one.

Pesticidally active compounds and other biologically active materials which may be included in, or used in conjuction with, the herbicidal compositions of the present invention, for example those hereinbefore mentioned, and which are acids, may, if desired, be utilized in the form of conventional derivatives, for example alkali metal and amine salts and esters.

According to a further feature of the present invention there is provided an article of manufacture comprising at least one of the benzimidazolyl derivatives of general formula (I) or, as is preferred, a herbicidal composition as hereinbefore described, and preferably a herbicidal concentrate which must be diluted before use, comprising at least one of the benzimidazolyl derivatives of general formula (I) within a container for the aforesaid derivative or derivatives of general formula (I), or a said herbicidal composition, and instructions physically associated with the aforesaid container setting out the manner in which the aforesaid derivative or derivatives of general formula (I) or herbicidal composition contained therein is so be used to control the growth of weeds. The containers will normally be of the types conventionally used for the storage of chemical substances which are solid at normal ambient temperatures and herbicidal compositions particularly in the form of concentrates, for example cans and drums of metal, which may be internally lacquered, and plastics materials, bottles or glass and plastics materials and, when the contents of the container is a solid, for example granular, herbicidal compositions, boxes, for example of cardboard, plastics materials and metal, or sacks. The containers will normally be of sufficient capacity to contain amounts of the benzimidazoyl derivative or herbicidal compositions sufficient to treat at least one acre of ground to control the growth of weeds therein but will not exceed a size which is convenient for conventional methods of handling. The instructions will be physically associated with the container, for example by being printed directly thereon or on a label or tag affixed thereto. The directions will normally indicate that the contents of the container, after dilution if necessary, are to be applied to control the growth of weeds at rates of application between 0.01kg and 20kg of active material per hectare in the manner and for the purposes hereinbefore described.

The following Examples illustrate herbicidal compositions according to the present invention:

**EXAMPLE C1**

A soluble concentrate was formed from :

| | | |
|---|---|---|
| Active ingredient (compound 1) | 20% | w/v |
| Potassium hydroxide solution 33% w/v | 10% | v/v |
| Tetrahydrofurfuryl alcohol (THFA) | 10% | v/v |
| Water | to 100 volumes. | |

by stirring THFA, active ingredient (compound 1) and 90% volume of water and slowly adding the potassium hydroxide solution until a steady pH 7-8 was obtained

then making up to volume with water.

Similarly soluble concentrates may be prepared as described above by replacing the benzimidazolyl (compound 1) with other compounds of general formula (I).

**EXAMPLE C2**

A wettable powder was formed from :

| | | |
|---|---|---|
| Active ingredient (compound 1) | 50% | w/w |

| | | |
|---|---|---|
| Sodium dodecylbenzene sulphonate | 3% | w/w |
| Sodium lignosulphate | 5% | w/w |
| Sodium formaldehyde alkylnaphthalene sulphonate | 2% | w/w |
| Microfine silicon dioxide | 3% | w/w and |
| China clay | 37% | w/w |

by blending the above ingredients together and grinding the mixture in an air jet mill.

Similar wettable powders may be prepared as described above by replacing the benzimidazolyl nicotinate (compound 1) with other compounds of general formula (I).

**EXAMPLE C3**

A water soluble powder was formed from :

| | | |
|---|---|---|
| Active ingredient (compound 1) | 50% | w/w |
| Sodium dodecylbenzenesulphonate | 1% | w/w |
| Microfine silicon dioxide | 2% | w/w |
| Sodium bicarbonate | 47% | w/w |

by mixing the above ingredients and grinding the above mixture in a hamermill.

Similar water soluble powders may be prepared as described above by replacing the benzimidazolyl nicotinate (compound 1) with other compounds of general formula (I).

Representative compounds of general formula (I) have been used in herbicidal applications according to the following procedures.

**METHOD OF USE OF HERBICIDAL COMPOUNDS:**

a) General

Appropriate quantities of the compounds used to treat the plants were dissolved in acetone to give solutions equivalent to application rates of up to 4000g test compound per hectare (g/ha). These solutions were applied from a standard laboratory herbicide sprayer delivering the equivalent of 290 litres of spray fluid per hectare.

b) Weed control : Pre-emergence

The seeds were sown in 70 mm square, 75 mm deep plastic pots in non-sterile soil. The quantities of seed per pot were as follows:-

| Weed species | Approx number of seeds/pot |
|---|---|
| 1) Broad-leafed weeds | |
| Abutilon theophrasti | 10 |
| Amaranthus retroflexus | 20 |
| Galium aparine | 10 |
| Ipomoea purpurea | 10 |
| Sinapis arvensis | 15 |
| Xanthium strumarium | 2. |
| 2) Grass weeds | |
| Alopecurus myosuroides | 15 |
| Avena fatua | 10 |
| Echinochloa crus-galli | 15 |
| Setaria viridis | 20. |
| 3) Sedges | |
| Cyperus esculentus | 3. |

| Crop | Approx number of seeds/pot |
|---|---|
| 1) Broad-leafed | |
| Cotton | 3 |
| Soya | 3. |
| 2) Grass | |
| Maize | 2 |
| Rice | 6 |
| Wheat | 6. |

The compounds of the invention were applied to the soil surface, containing the seeds, as described in (a). A single pot of each crop and each weed was allocated to each treatment, with unsprayed controls and controls sprayed with acetone alone.

After treatment the pots were placed on capillary matting kept in a glass house, and watered overhead. Visual assessment of crop damage was made 20-24 days after spraying. The results were expressed as the percentage reduction in growth or damage to the crop or weeds, in comparison with the plants in the control pots.

c) Weed control : Post-emergence

The weeds and crops were sown directly into John Innes potting compost in 75 mm deep, 70 mm square

pots except for Amaranthus which was pricked out at the seedling stage and transferred to the pots one week before spraying. The plants were then grown in the greenhouse until ready for spraying with the compounds used to treat the plants. The number of plants per pot were as follows:-

1) Broad leafed weeds

| Weed species | Number of plants per pot | Growth stage |
|---|---|---|
| Abutilon theophrasti | 3 | 1-2 leaves |
| Amaranthus retroflexus | 4 | 1-2 leaves |
| Galium aparine | 3 | $1^{st}$ whorl |
| Ipomoea purpurea | 3 | 1-2 leaves |
| Sinapis arvensis | 4 | 2 leaves |
| Xanthium strumarium | 1 | 2-3 leaves. |

2) Grass weeds

| Weed species | Number of plants per pot | Growth stage |
|---|---|---|
| Alopecurus myosuroides | 8-12 | 1-2 leaves |
| Avena fatua | 12-18 | 1-2 leaves |
| Echinochloa crus-galli | 4 | 2-3 leaves |
| Setaria viridis | 15-25 | 1-2 leaves. |

3) Sedges

| Weed species | Number of plants per pot | Growth stage |
|---|---|---|
| Cyperus esculentus | 3 | 3 leaves. |

1) Broad leafed

| Crops | Number of plants per pot | Growth stage |
|---|---|---|
| Cotton | 2 | 1 leaf |
| Soya | 2 | 2 leaves. |

2) Grass

| Crops | Number of plants per pot | Growth stage |
|---|---|---|
| Maize | 2 | 2-3 leaves |
| Rice | 4 | 2-3 leaves |
| Wheat | 5 | 2-3 leaves. |

The compounds used to treat the plants were applied to the plants as described in (a). A single pot of each crop and weed species was allocated to each treatment, with unsprayed controls and controls sprayed with acetone alone.

After treatment the pots were placed on capillary matting in a glass house, and watered overhead once after 24 hours and then by controlled sub-irrigation. Visual assessment of crop damage and weed control was made 20-24 days after spraying. The results were expressed as the percentage reduction in growth or damage to the crop or weeds, in comparison with the plants in the control pots.

The compounds of the invention used at 4000 g/ha or less, have shown an excellent level of herbicidal activity in the foregoing experiments, giving 90% reduction in growth of one or more weed species when applied pre- or post- emergence,combined with tolerance on one or more crops.

When applied pre-emergence at 4000 g/ha compound 47 gave at least 90% reduction in growth of one or more weed species..

When applied post-emergence at 4000 g/ha compounds 1,2,3,4,5,9, 11,12,13,14,15,16,17,18,19,20, 21,22,23,24,25,26,27,28,33,34,35, 36,37,38,39,40,41 and 45 gave at least 90% reduction in growth of one or more weed species.

When applied pre-emergence at 1000 g/ha compounds 8,10 and 25 gave at least 90% reduction in growth of one or more weed species.

When applied post-emergence at 1000 g/ha compounds 1,4,5,6,7, 11,12,13,16,17,18,19,20,21,22, 23,24,26,27,28,34,35,36,37,38,40, 41,44,46,48 and 49 gave at least 90% reduction in growth of one or more weed species.

## Claims

**1.** A benzimidazolyl derivative of general formula I:-

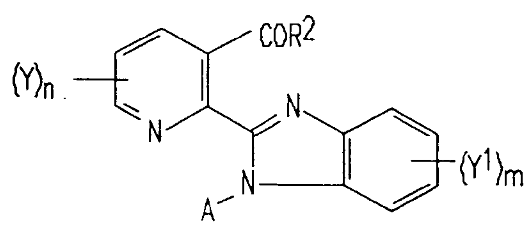

wherein

Y represents:-

a straight- or branched- chain alkyl, alkenyl or alkynyl group containing up to eight carbon atoms which is optionally substituted by one or more $R^1$ groups which may be the same or different; or

a cycloalkyl group containing from 3 to 6 carbon atoms optionally substituted by one or more $R^1$ groups which may be the same or different; or a group selected from -SR, -OR, halogen, aryl or aralkyl;

$Y^1$ represents:-

a straight- or branched- chain alkyl, alkenyl or alkynyl group containing up to eight carbon atoms which is optionally substituted by one or more $R^1$ groups which may be the same or different; or

a cycloalkyl group containing from 3 to 6 carbon atoms optionally substituted by one or more $R^1$ groups which may be the same or different; or a group selected from -SR, -OR, halogen, aryl, aralkyl, O-aryl or $-NR^7R^8$;

$R^2$ represents:-

a group -OH or $-NR^7R^8$;

or -X-M, where X represents oxygen or sulphur, and

M represents:-

a straight- or branched- chain alkyl group containing up to eight carbon atoms optionally substituted by one or more $R^1$ groups which may be the same or different; or

a cycloalkyl group containing from 3 to 6 carbon atoms optionally substituted by one or more $R^1$ groups which may be the same or different; or a group selected from aryl and aralkyl; or

a group 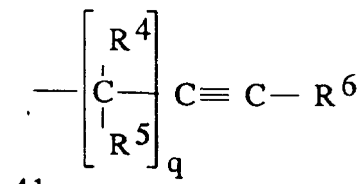 ; or

a group 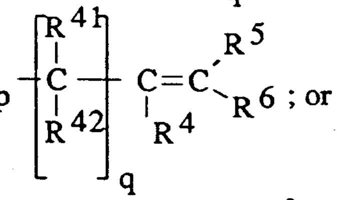 ; or

a group 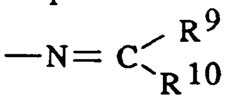 ;

A represents a group $-SO_2NR^7R^8$;
R represents;-

a straight- or branched- chain alkyl group containing up to eight carbon atoms optionally substituted by one or more groups $R^1$ which may be the same or different; or

a cycloalkyl group containing from 3 to 6 carbon atoms optionally substituted by one or more groups $R^3$ which may be the same or different;

$R^1$ represents:-

a cycloalkyl group containing from 3 to 6 carbon atoms; or

a group selected from $-OR^3$, $-SR^3$, halogen, $R^3$ or O-aryl;

$R^3$ represents a straight- or branched- chain alkyl group containing up to 6 carbon atoms optionally substituted by one or more halogen atoms which may be the same or different;

$R^4$, $R^{41}$, $R^{42}$ and $R^5$, which may be the same or different, each represent hydrogen or a straight- or branched- chain alkyl group containing up to 6 carbon atoms optionally substituted by one or more halogen atoms which may be the same or different; or aryl;

$R^6$ represents a group selected from $R^4$ or aralkyl;

$R^7$ and $R^8$, which may be the same or different, each represent:-

a hydrogen atom, or

a group selected from R, $-OR^3$, $-SR^3$, halogen, $R^3$, O-aryl, aryl or aralkyl; or $R^7$ and $R^8$ may form together with the nitrogen to which they are attached a heterocycle containing from 3 to 6 carbon atoms in the ring and zero, 1 or 2 additional heteroatoms in the ring selected from nitrogen, oxygen and sulphur;

$R^9$ and $R^{10}$, which may be the same or different, each represent:

a hydrogen atom, or

a straight- or branched- chain alkyl group containing up to eight carbon atoms optionally substituted by one or more groups, which may be the same or different, selected form halogen, -OR or $-S(O)_rR$, where r is zero, 1 or 2; or

a phenyl group optionally substituted by from one to four groups, which may be the same or different, selected from nitro, R, $-NR^4R^5$, halogen and $-S(O)_rR$; or

a 5 or 6 membered heterocycle containing from 3 to 5 carbon atoms in the ring and one or more heteroatoms in the ring selected from nitrogen, sulphur or oxygen, optionally substituted by one or more groups $R^1$ which may be the same or different;

'aryl' represents:-

a phenyl group optionally substituted by from 1 to 4 groups which may be the same or different selected from $-OR^3$, $-SR^3$, halogen or $R^3$; or

a 5 or 6 membered heterocycle containing from 3 to 5 carbon atoms in the ring and one or more heteroatoms in the ring selected from nitrogen, sulphur or oxygen, optionally substituted by one or more groups, which may be the same or different, selected from $-OR^3$, $-SR^3$, halogen or $R^3$;

'aralkyl' represents a group $-(CR^4R^5)_p$-aryl;

m represents an integer from 1 to 4, the groups $Y^1$ being the same or different when m is greater than 1;

n represents one or two;

when n represents 2, two groups Y in the 5- and 6- positions of the pyridine ring together from an unsubstituted fused phenyl ring;

p represents one or two;

q represents one or two; and
where m represents an integer greater than 1, two groups $Y^1$ selected from -SR, -OR and straight- or branched- chain alkyl groups containing up to 8 carbon atoms optionally substituted by one or more groups $R^1$ which may be the same or different, together in the 4,5 position or the 5,6 position can, with the carbon atoms to which they are attached, form an aliphatic ring having 5 or 6 atoms in the ring and having not more than 2 heteroatoms in the ring selected from oxygen and sulphur;
and agriculturally acceptable salts thereof.

2. A compound according to claim 1 wherein n represents one.

3. A compound according to claim 1 wherein
n represents 2 and two groups Y in the 5- and 6- positions of the pyridine ring together with the carbon atoms to which they are attached form an unsubstituted fused phenyl ring;
A represents $-SO_2NMe_2$;
$Y^1$ represents:-
a halogen atom, a group -OR, or a straight- or branched- chain alkyl group containing up to 4 carbon atoms optionally substited by one or more halogen atoms ;
m represents one or two; and
R represents a straight- or branched- chain alkyl group containing up to 4 carbon atoms optionally substituted by one or more halogen atoms.

4. A compound according to claim 1 to 2 wherein:
(a) A represents $-SO_2NM_2$; and/or
(b) $R^2$ represents -OH or XM; and/or
(c) Y represents an alkyl group containing one or two carbon atoms optionally substituted by one or more halogen atoms; and/or
(d) $Y^1$ represents -OR, halogen or $-NR^7R^8$; and/or
(e) m represents one or two; and/or
(f) $R^7$ and $R^8$, which may be the same or different, each represent a straight- or branched-chain alkyl group containing up to 4 carbon atoms optionally substituted by one or more atoms which may be the same or different selected form fluorine or chlorine; and/or
(g) X represents oxygen; and/or
(h) M represents $-CH_2C{\equiv}CH$ or $CH_2CH{=}CH_2$; and/or
(i) the group or groups $Y^1$ occupy the 4- and/or 5 position of the benzimidazole ring; and/or
(j) n represents one.

5. A compound according to any one of claims 1 to 3 wherein $Y^1$ represents a straight- or branched- chain alkyl group containing up to 4 carbon atoms.

6. A compound according to claim, 1,2,4 or 5 wherein
A represents $-SO_2NMe_2$;
Y represents methyl or ethyl, or two groups Y in the 5- and 6- positions of the pyridine ring together with the carbon atoms to which they are attached form an unsubstituted fused phenyl ring;
$R^2$ represents -OH or -X-M ;
X represents oxygen;
M represents 2-propynyl, methyl or an isopropylammonium or potassium cation;
$Y^1$ represents chlorine, methyl, methoxy, ethoxy, n-propyloxy or isopropyloxy;
m represents one or two; and
n represents one or two.

7. A compound according to claim 1 which is
2-propynyl 2-[1-(N,N-dimethylsulphamoyl)-4-methoxy-benzimidazol-2-yl]-6-methylpyridine-3-carboxylate;
2-propynyl 2-[1-(N,N-dimethylsulphamoyl)-4-ethoxy-benzimidazol-2-yl]-6-methylpyridine-3-carboxylate;
2-propynyl 2-[1-(N,N-dimethylsulphamoyl)-5-chloro-benzimidazol-2-yl]-6-methylpyridine-3-carboxylate;
2-propynyl 2-[1-(N,N-dimethylsulphamoyl)-5(6)-methylbenzimidazol-2-yl]-6-methylpyridine-3-carboxylate;

2-propynyl 2-[1-(N,N-dimethylsulphamoyl)-5(6)-methylbenzimidazol-2-yl]-5-methylpyridine-3-carboxylate;
2-propynyl 2-[1-(N,N-dimethylsulphamoyl)-4-methoxy-benzimidazol-2-yl]-5-methylpyridine-3-carboxylate;
2-propynyl 2-[1-(N,N-dimethylsulphamoyl)-5-chloro-benzimidazol-2-yl]-5-methylpyridine-3-carboxylate;
2-[1-(N,N-dimethylsulphamoyl)-4-methoxybenzimidazol-2-yl]-6-methylpyridine-3-carboxylic acid;
2-[1-(N,N-dimethylsulphamoyl)-5-fluorobenzimidazol-2-yl]-6-methylpyridine-3-carboxylic acid;
2-[1-(N,N-dimethylsulphamoyl)-4-ethoxybenzimidazol-2-yl]-6-methylpyridine-3-carboxylic acid;
2-[1-(N,N-dimethylsulphamoyl)-4,5-dimethylbenzimidazol-2-yl]-6-methylpyridine-3-carboxylic acid;
2-[1-(N,N-dimethylsulphamoyl)-5-chlorobenzimidazol-2-yl]-6-methylpyridine-3-carboxylic acid;
2-[1-(N,N-dimethylsulphamoyl)-5(6)-methoxybenzimidazol-2-yl]-6-methylpyridine-3-carboxylic acid;
2-[1-(N,N-dimethylsulphamoyl)-4-methylbenzimidazol-2-yl]-6-methylpyridine-3-carboxylic acid;
2-[1-(N,N-dimethylsulphamoyl)-4-*n*-propyloxy-benzimidazol-2-yl]-6-methylpyridine-3-carboxylic acid;
2-[1-(N,N-dimethylsulphamoyl)-4-*i*-propyloxybenzimidazol-2-yl]-6-methylpyridine-3-carboxylic acid;
2-[1-(N,N-dimethylsulphamoyl)-4-chlorobenzimidazol-2-yl]-6-methylpyridine-3-carboxylic acid;
2-[1-(N,N-dimethylsulphamoyl)-5(6)-methylbenzimidazol-2-yl]-5-ethylpyridine-3-carboxylic acid;
2-[1-(N,N-dimethylsulphamoyl)-5(6)-methoxybenzimidazol-2-yl]-5-ethylpyridine-3-carboxylic acid;
2-[1-(N,N-dimethylsulphamoyl)-5-chlorobenzimidazol-2-yl]-5-ethylpyridine-3-carboxylic acid;
2-[1-(N,N-dimethylsulphamoyl)-4-methoxybenzimidazol-2-yl]-5-ethylpyridine-3-carboxylic acid;
2-[1-(N,N-dimethylsulphamoyl)-4-methoxybenzimidazol-2-yl]-5-ethylpyridine-3-carboxylic acid;
2-[1-(N,N-dimethylsulphamoyl)-4-ethoxybenzimidazol-2-yl]-5-ethylpyridine-3-carboxylic acid;
2-[1-(N,N-dimethylsulphamoyl)-4-n-propyloxy-benzimidazol-2-yl]-5-ethylpyridine-3-carboxylic acid;
2-[1-(N,N-dimethylsulphamoyl)-4-methoxybenzimidazol-2-yl]-5-methylpyridine-3-carboxylic acid;
2-[1-(N,N-dimethylsulphamoyl)-5(6)-methylbenzimidazol-2-yl]-5-methylpyridine-3-carboxylic acid;
2-[1-(N,N-dimethylsulphamoyl)-4-methylbenzimidazol-2-yl]-5-methylpyridine-3-carboxylic acid;
2-[1-(N,N-dimethylsulphamoyl)-5-chlorobenzimidazol-2-yl]-5-methylpyridine-3-carboxylic acid;
2-[1-(N,N-dimethylsulphamoyl)-4-*n*-propyloxybenzimidazol-2-yl]-5-methylpyridine-3-carboxylic acid;
2-[1-(N,N-dimethylsulphamoyl)-4-ethoxybenzimidazol-2-yl]-5-methylpyridine-3-carboxylic acid;
2-[1-(N,N-dimethylsulphamoyl)-4-*i*-propyloxybenzimidazol-2-yl]-5-methylpyridine-3-carboxylic acid;
2-[1-(N,N-dimethylsulphamoyl)-4-*i*-propyloxybenzimidazol-2-yl]-5-ethylpyridine-3-carboxylic acid;
isopropylammonium 2-[1-(N,N-dimethylsulphamoyl)-5-chlorobenzimidazol-2-yl]-6-methylpyridine-3-carboxylate;
potassium 2-[1-(N,N-dimethylsulphamoyl)-4-methylbenzimidazol-2-yl]-5-methylpyridine-3-carboxylate;
isopropylammonium 2-[1-(N,N-dimethylsulphamoyl)-4-methyl-benzimidazol-2-yl]-5-methylpyridine - 3-carboxylate;
potassium 2-[1-(N,N-dimethylsulphamoyl)-5-chloro-benzimidazol-2-yl]-5-methylpyridine-3-carboxylate;
isopropylammonium 2-[1-(N,N-dimethylsulphamoyl)-5-chloro-benzimidazol-2-yl]-5-methylpyridine-3-carboxylate;
2-[1-(N,N-dimethylsulphamoyl)-4-methoxybenzimidazol-2-yl]quinoline-3-carboxylic acid;
2-[1-(N,N-dimethylsulphamoyl)-4-ethoxybenzimidazol-2-yl]quinoline-3-carboxylic acid;
2-[1-(N,N-dimethylsulphamoyl)-4,5-dimethylbenzimidazol-2-yl]quinoline-3-carboxylic acid;
2-[1-(N,N-dimethylsulphamoyl)-5(6)-methylbenzimidazol-2-yl]quinoline-3-carboxylic acid;
2-[1-(N,N-dimethylsulphamoyl)-4-*i*-propyloxybenzimidazol-2-yl]quinoline-3-carboxylic acid;
2-[1-(N,N-dimethylsulphamoyl)-4-methylbenzimidazol-2-yl]quinoline-3-carboxylic acid;
2-[1-(N,N-dimethylsulphamoyl)-5-chlorobenzimidazol-2-yl]quinoline-3-carboxylic acid;
2-propynyl 2[1-(N,N-dimethylsulphamoyl)-5(6)-methylbenzimidazol-2-yl]quinoline-3-carboxylate.
methyl 2-[1-(N,N-dimethylsulphamoyl)-4-methoxy-benzimidazol-2-yl]-6-methylpyridine-3-carboxylate;
methyl 2-[1-(N,N-dimethylsulphamoyl)-5-chloro-benzimidazol-2-yl]-6-methylpyridine-3-carboxylate;
methyl 2-[1-(N,N-dimethylsulphamoyl)-4-methoxy-benzimidazol-2-yl]quinoline-3-carboxylate; or
methyl 2-[1-(N,N-dimethylsulphamoyl)-4-ethoxy-benzimidazol-2-yl]quinoline-3-carboxylate.

8. A process for the preparation of a compound of general formula I according to claim 1 or 2 which comprises:
a) where $R^2$ is a -XM or $-NR^7R^8$ radical, the reaction of a sulphamoyl chloride of the formula $ClSO_2NR^7R^8$ with a compound of formula

(Ia):

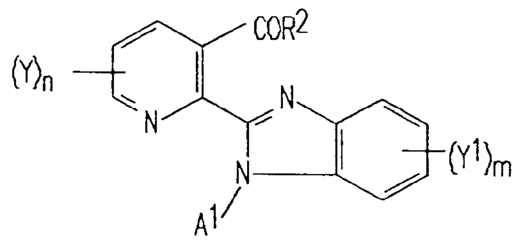

Ia

in which $A^1$ is the hydrogen atom, $R^2$ is an -XM or $NR^7R^8$ radical and the other symbols are as defined in claim 1, in the presence of an acid acceptor;

b) where $R^2$ represents -OH, the hydrolysis of a compound of formula (I) in which $R^2$ represents the group -XM;

c) where m represents 1 or 2, one of the groups $Y^1$ represents a group -OR which occupies the 4- or 5- position of the benzimidazole ring, $R^2$ represents a group -X-M wherein X is oxygen and M represents a straight- or branched- chain alkyl group containing up to 8 carbon atoms substituted by one or more $R^1$ groups which may be the same or different, or a cycloalkyl group containing from 3 to 6 carbon atoms optionally substituted by one or more $R^1$ groups which may be the same or different and the group Y is as defined in claim 1 excluding an alkenyl or alkynyl group or chlorine, bromine or iodine, the reaction of a hydroxybenzimidazole of formula (XIV):

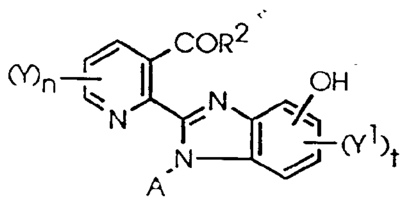

(XIV)

wherein t is 0 or 1 and the hydroxy group occupies the 4- or 5 position of the benzimidazole ring, with a compound R-L where L represents a leaving group and the other symbols are as defined in claim 1, to convert the hydroxy group in benzimidazole ring into a group -OR;

optionally followed by the conversion of a compound of formula (I) thus obtained into a salt thereof.

**9.** A herbicidal composition which comprises as active ingredient a herbicidally effective amount of a benzimidazoyl derivative of general formula (I) according to any one of claims 1 to 7 or an agriculturally acceptable salt thereof, in association with an agriculturally acceptable diluent or carrier and/or surface active agent.

**10.** A herbicidal composition according to claim 9 which comprises 0.05 to 90% by weight of active ingredient.

**11.** A herbicidal composition according to claim 9 or 10 which is in liquid form and contains from 0.05 to 25% of surface-active agent.

**12.** A herbicidal compositions according to claim 9, 10 or 11 in the form of an aqueous suspension concentrate, a wettable powder, a water soluble or water dispersible powder, a liquid water soluble concentrate, a liquid emulsifiable suspension concentrate, a granule or an emulsifiable concentrate.

**13.** A method for controlling the growth of weeds at a locus which comprises applying to the locus a herbicidally effective amount of a benzimidazolyl derivative of general formula (I) according to any one of claims 1 to 7 or an agriculturally acceptable salt thereof.

**14.** A method according to claim 13 in which the locus is an area used, or to be used, for growing of crops and the compound is applied at an application rate from 0.01 kg to 4.0 kg per hectare.

**15.** A method according to claim 13 in which the locus is an area which is not a crop-growing area and the compound is applied at an application rate from 1.0 kg to 20.0 kg per hectare.

**16.** A compound of formula (Ia) as defined in claim 8.

European Patent Office

# EUROPEAN SEARCH REPORT

Application Number

EP 92 30 3197

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | FR-A-2 021 657 (AGFA-GEVAERT AKTIENGESELLSCHAFT)<br>--- | | C07D401/04<br>A01N43/50 |
| E | EP-A-0 429 372 (RHONE-POULENC AGROCHIMIE)<br>* the whole document *<br>----- | 1-16 | C07D491/04<br>C07D495/04<br>A01N43/40<br>A01N43/90<br>A01N43/42 |
| | | | TECHNICAL FIELDS SEARCHED (Int. Cl.5) |
| | | | C07D<br>A01N |

The present search report has been drawn up for all claims

| Place of search | Date of completion of the search | Examiner |
|---|---|---|
| THE HAGUE | 24 JUNE 1992 | DE BUYSER I.A.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)